(19)

Europäisches Patentamt
European Patent Office
Office européen des brevets

(11) **EP 3 498 314 A1**

(12) **EUROPEAN PATENT APPLICATION**
published in accordance with Art. 153(4) EPC

(43) Date of publication:
**19.06.2019 Bulletin 2019/25**

(51) Int Cl.:
**A61M 1/02** (2006.01)

(21) Application number: **17839192.6**

(22) Date of filing: **21.07.2017**

(86) International application number:
**PCT/JP2017/026482**

(87) International publication number:
**WO 2018/030119 (15.02.2018 Gazette 2018/07)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **10.08.2016 JP 2016157115**
**12.08.2016 JP 2016158488**
**27.12.2016 JP 2016252219**
**12.01.2017 JP 2017003042**

(71) Applicant: **Kawasumi Laboratories, Inc.**
**Saiki-shi, Oita 876-0121 (JP)**

(72) Inventors:
• **WATANABE Masatoshi**
**Bungo-Ono-shi**
**Oita 879-7153 (JP)**
• **GOTOH Shoichi**
**Bungo-Ono-shi**
**Oita 879-7153 (JP)**
• **NAKAYA Seiichi**
**Bungo-Ono-shi**
**Oita 879-7153 (JP)**

(74) Representative: **Dennemeyer & Associates S.A.**
**Postfach 70 04 25**
**81304 München (DE)**

(54) **BLOOD TREATMENT INSTRUMENT**

(57) To provide a blood processing device, in which a blood processing member can secure an effective filtration area substantially two times greater than those of conventional ones and therefore it becomes possible to largely reduce the filtration time. [Solution] A first blood processing member 5U formed by stacking multiple non-woven fabrics each having a predetermined thickness and a second blood processing member 5D formed by stacking multiple non-woven fabrics each having a predetermined thickness are arranged in a first housing 2/U and a second housing 2/D, respectively. The first blood processing member 5U and the second blood processing member 5D are arranged in such a manner that inside filtration sections 5FI/U and 5FI/D face each other. A first blood chamber 2.1 is formed between the inside filtration sections 5FI/U and 5FI/D, and a second blood chamber 2.2 is formed between the inner periphery of the first housing 2/U and an outside filtration section 5FO/U of the first blood processing member 5/U and between the inner periphery of the second housing 2/D and an outside filtration section 5FO/D of the second blood processing member 5D. A blood inlet port 71 is formed so as to be communicated with the first blood chamber 2.1 (a blood inflow chamber 2IR), and a blood discharge port 7O is formed so as to be communicated with the second blood chamber 2.2 (a blood outflow chambers 20R/U and 20R/D).

FIG. 9

## Description

Technical Field

The present invention relates to a blood processing device used in medical appliances such as a blood collection device and a blood circuit used for hemodialysis, blood purification, and the like.

Background Art

In PTL 1 and PTL 2, the present applicant disclosed inventions of a blood processing device (leukocyte removal filter) constituted with a flexible housing and nonwoven cloth as a blood processing member (blood filter) installed in the interior of the housing.

Furthermore, PTL 3 discloses an invention of a blood processing device (leukocyte remover) in which a plurality of ribs 23 arranged substantially in parallel with depressions and projections having a height difference of 0.2 to 2 mm (specifically, from one end to the other end (in other words, a blood flow direction)) are formed on an inner surface of a flexible housing (bag-like housing made of a soft resin) facing the surface of a outflow side blood chamber of a blood processing member (leukocyte removal filter member) such that a blood filtration speed is hardly reduced and adhesion failure of the housing does not occur (see paragraphs [0028] to [0033]).

In PTL 3, a sponge-like porous polyurethane material is used as the blood processing member, the number of sheets of the porous material to be laminated is set to be 6 (10 at most), and the outermost peripheral portion of the blood processing member is fixed to the flexible housing through an outer frame (sheet). In this way, a blood flow path between the outer frame sheet and the inner surface of the flexible housing is formed in an inner peripheral portion of the flexible housing, and accordingly, the circulation of blood in the inner peripheral portion of the flexible housing is improved, and blood is prevented from remaining in the inner peripheral portion.

In the invention disclosed in PTL 4, nonwoven cloth is fixed to a flexible housing by using an inner side sealing portion (first sealing portion) and an outer side sealing portion (second sealing portion) without using the outer frame (sheet) described above, and a blood inlet (upper side) and a blood outlet (lower side) are mounted on a side deeper than where the inner side sealing portion is located.

Citation List

Patent Literature

    PTL 1
Japanese Patent No. 3014916 (CLAIMS and FIGS.

1 to 10)
PTL 2
Japanese Patent No. 3049182 (CLAIMS and FIGS. 1 to 6)
PTL 3
Japanese Patent No. 3758853 (CLAIMS, paragraphs [0028] to [0033], and FIGS. 1 to 10)
PTL 4
Japanese Patent No. 4038547 (CLAIMS and FIGS. 2, 3, and 4)

Summary of Invention

Technical Problem

In the blood processing devices described in PTLs 1 to 4, a flexible housing is used. Therefore, as blood filtration progresses, the internal pressure of the internal space of the flexible housing on the blood outflow chamber side becomes negative. As a result, the housing tends to be crushed, and the blood flow path in the vicinity of a blood outflow port tends to be blocked. Accordingly, it is apprehended that the blood filtration speed may be reduced.

In contrast, in the invention described in PTL 3, depressions and projections having a height difference of up to 2 mm are formed on the inner surface of the flexible housing (bag-like housing made of a soft resin). However, forming these types of depressions and projections is not enough to effectively prevent the blood flow path in the vicinity of the blood outflow port from being blocked due to negative pressure.

In the invention described in PTL 4, a sheet on the blood inlet side and a sheet on the blood outlet side in the flexible housing have to be perforated so as to install the blood inlet and the blood outlet. Therefore, unfortunately, the processing of the invention in PTL 4 is more difficult than that of PTL 3.

Solution to Problem

The inventors of this specification have repeatedly conducted keen studies for the purpose of solving the problems mentioned above and thus has come to the following invention.

    [1] The present invention provides a blood processing device (1, 1', 101), comprising:

        a housing (2);
        a blood inlet section (71, 71') disposed on a proximal end (PE) side of the housing (2);
        a blood outlet section (7O, 7O') disposed on a distal end (DE) side of the housing (2);
        an upper portion-side first blood processing member [5/U, (5/PEU, 5/DEU)] which is disposed in the housing (2) and formed by laminating a plurality of sheets of nonwoven cloth having a predetermined thickness; and

a lower portion-side second blood processing member [5/D, (5/PED, 5/DED)] which is disposed in the housing (2) and formed by laminating a plurality of sheets of nonwoven cloth having a predetermined thickness,

wherein the upper portion-side first blood processing member [5/U, (5/PEU, 5/DEU)] and the lower portion-side second blood processing member [5/D, (5/PED, 5/DED)] each have an inner side filtration section (5FI, 5FI') and an outer side filtration section (5FO, 5FO'), wherein the upper portion-side first blood processing member [5/U, (5/PEU, 5/DEU)] and the lower portion-side second blood processing member [5/D, (5/PED, 5/DED)] are disposed such that the inner side filtration sections (5FI, 5FI') thereof face each other,

an inner side first blood chamber (2.1) is formed between the inner side filtration sections (5FI, 5FI'), and

an outer side second blood chamber (2.2) is formed between an inner periphery of the housing (2) and the outer side filtration sections (5FO, 5FO') of the upper portion-side first blood processing member [5/U, (5/PEU, 5/DEU)] and the lower portion-side second blood processing member [5/D, (5/PED, 5/DED)].

[2] The present invention provides the blood processing device (1, 1', 101) described in [1], wherein the inner side first blood chamber (2.1) functions as a blood inflow chamber (2IR) or a blood outflow chamber (2OR'), and

the outer side second blood chamber (2.2) functions as a blood outflow chamber (2OR) or a blood inflow chamber (2IR').

Herein, for clarifying the technical contents of [1] and [2] described above, the following definitions are given just in case. In the case of blood processing device 1 (blood processing device 1'), inner side first blood chamber (2.1) is blood inflow chamber (2IR) (in the case of blood processing device 1) or blood outflow chamber (2OR') (in the case of blood processing device 1') formed of a space which is between and surrounded by inner side filtration sections (5FI and 5FI'), which face each other, of upper portion-side first blood processing member (5/U) and lower portion-side second blood processing member (5/D).

Outer side second blood chamber (2.2) is blood outflow chamber (2OR) (in the case of blood processing device 1) or blood inflow chamber (2IR') (in the case of blood processing device 1') formed of a space that is between and surrounded by the outsides (outer peripheries) [outer side filtration sections (5FO and 5FO')], which face each other, of the blood processing members including upper portion-side first blood processing member (5/U) and lower portion-side second blood processing member (5/D) and the inside (inner periphery) of housing 2.

In the case of blood processing device 101, inner side first blood chamber (2.1) is constituted with proximal end-side first blood inflow chamber (2IR/PE) and distal end-side second blood inflow chamber (2IR/DE), and outer side second blood chamber (2.2) is constituted with proximal end-side first blood outflow chamber 2OR/PE and distal end-side second blood outflow chamber 2OR/DE.

[3] The present invention provides the blood processing device (1, 1', 101) described in [1], wherein an upper portion-side first housing (2/U) and a lower portion-side second housing (2/D) are formed by processing a thick flexible sheet into a three-dimensional shape,

the upper portion-side first housing (2/U) and the lower portion-side second housing (2/D) are three-dimensionally formed such that an interior (2I) thereof rises from an outer edge portion (2F), and

a plurality of projections (T) are provided in the interior (2I) of the second housing (2/D) or in the interior (2I) of the second housing (2/D) and the first housing (2/U).

Note that, the blood processing device described in [3] makes possible a faster (less) filtering time by disposing the blood processing member 5 in the housing (2/U, 2/D) in the blood processing device(1, 1', 101) including the blood processing member 5 defined in [1], the housing (2/U, 2/D) including a thickness, three-dimensional processing, and projections (T), which are characteristics of blood processing device 201 to be described, hereinafter.

[4] The present invention provides the blood processing device (1, 1') described in [1] or [2], wherein the blood inlet section (71, 71') is disposed on the proximal end (PE) side of the upper portion-side first housing (2/U) and the lower portion-side second housing (2/D),

the blood outlet section (7O, 7O') is disposed on the distal end (DE) side of the first housing (2/U) and the second housing (2/D), and

(i) in a case where the blood inlet section (7I) is formed to communicate with the inner side first blood chamber (2.1), the blood outlet section (7O) is formed to communicate with the outer side second blood chamber (2.2), the inner side first blood chamber (2.1) functions as a blood inflow chamber (2IR/U, 2IR/D), and the outer side second blood chamber (2.2) functions as a blood outflow chamber (2OR/U, 2OR/D), or

(ii) in a case where the blood inlet section (7I') is formed to communicate with the outer side second blood chamber (2.2), the blood outlet section (7O') is formed to communicate with the inner side first blood chamber (2.1), the outer side second blood chamber (2.2) functions as a blood inflow chamber (2IR//U, 2IR'/D), and the inner side first blood chamber (2.1) functions as a blood outflow chamber (2OR').

[5] The present invention provides the blood processing device (1, 1') described in [4], wherein an outer edge of the first blood processing member (5/U) on an upper portion side is fixed to an inner edge of a pair of upper and lower first outer frame sheets (3/UU, 3/UD) on an upper portion side in a state where the outer edge is held by the first outer frame sheets (3/UU, 3/UD),
an inner edge of the first flexible housing (2/U) is fixed to an outer edge of the first outer frame sheets (3/UU, 3/UD),
an outer edge of the second blood processing member (5D) on a lower portion side is fixed to an inner edge of a pair of upper and lower second outer frame sheets (3/DU, 3/DD) on a lower portion side in a state where the outer edge is held by the second outer frame sheets (3/DU and 3/DD), and
an inner edge of the second flexible housing (2/D) is fixed to an outer edge of the second outer frame sheets (3/DU, 3/DD).

[6] The present invention provides the blood processing device (1, 1') described in [5],
wherein in the pair of upper and lower first outer frame sheets (3/UU, 3/UD) on upper portion side and the pair of upper and lower second outer frame sheets (3/DU, 3/DD) on the lower portion side, an upper portion-side first lengthened section (3L/U) and a lower portion-side second lengthened section (3L/D) are formed on the proximal end (PE) side or the distal end (DE) side, and

> (i) on the proximal end (PE) side where the blood inlet section (7I) is fixed, from an upper portion (U) side to a lower portion (D) side, the upper portion-side first housing (2/U), the upper portion-side first lengthened section (3L/U), the blood inlet section (7I), the lower portion-side second lengthened section (3L/D), and the lower portion-side second housing (2/D) are fixed in this order, and
> on the distal end (DE) side where the blood outlet section (7O) is fixed, from the upper portion (U) side to the lower portion (D) side, the upper portion-side first housing (2/U), the blood outlet section (7O), and the lower portion-side second housing (2/D) are fixed in this order, or
> (ii) on the proximal end (PE) side where the blood inlet section (7I') is fixed, from the upper portion (U) side to the lower portion (D) side, the upper portion-side first housing (2/U), the blood inlet section (7I'), and the lower portion-side second housing (2/D) are fixed in this order, and

on the distal end (DE) side where the blood outlet section (7O') is fixed, from the upper portion (U) side to the lower portion (D) side, the upper portion-side first lengthened section (3L/U), the upper portion-side first housing (2/U), the blood outlet section (7O'), the lower portion-side second lengthened section (3L/D), and the lower portion-side second housing (2/D) are fixed in this order.

[7] The present invention provides the blood processing device (1, 1') as described in [4],
wherein an inlet side hole (8I) is formed at a position closer to the proximal end (PE) side than an inlet opening (7IO) on an extreme distal end (DE) side of the blood inlet section (7I), and
the inlet side hole (8I) is formed in the vicinity of the proximal end (PE) side of an inner side filtration section (5FI/U, 5FI/D) of the blood processing member (5/U, 5/D), or
an outlet side hole (8O) is formed at a position closer to the distal end (DE) side than an outlet opening (7OO) on an extreme proximal end (PE) side of the blood outlet section (7O'), and
the outlet side hole (8O) is formed in the vicinity of the distal end (DE) side of an inner side filtration section (5FI'/U, 5FI'/D) of the blood processing member (5/U, 5/D).

[8] The present invention provides the blood processing device (101) described in [1],
wherein a pair of upper and lower proximal end-side first blood processing members (5/PEU, PED) formed by laminating a plurality of sheets of nonwoven cloth having a predetermined thickness are disposed in a proximal end (PE) side (PE-side Area) in the housing (2), and
a pair of upper and lower distal end-side second blood processing members (5/DEU, DED) formed by laminating a plurality of sheets of nonwoven cloth having a predetermined thickness are disposed in a distal end (DE) side (DE-side Area) in the housing (2), wherein
the pair of upper and lower proximal end-side first blood processing members (5/PEU, PED) and the pair of upper and lower distal end-side second blood processing members (5/DEU, DED) each have the inner side filtration section (5FI) and the outer side filtration section (5FO), wherein
the pair of upper and lower proximal end-side first blood processing members (5/PEU, PED) and the pair of upper and lower distal end-side second blood processing members (5/DEU, DED) are disposed such that the inner side filtration sections (5FI) thereof face each other,
a proximal end-side first blood inflow chamber (2IR/PE) is formed between the inner side filtration sections (5FI), which face each other, of the pair of upper and lower proximal end-side first blood processing members (5/PEU, PED),

a distal end-side second blood inflow chamber (2IR/DE) is formed between the inner side filtration sections (5FI), which face each other, of the pair of upper and lower distal end-side second blood processing members (5/DEU, DED),

a proximal end-side blood outflow chamber (2OR/PE) and a distal end-side blood outflow chamber (2OR/DE) are formed between the inner periphery of the housing (2) and the outer side filtration sections (5FO) of the pair of upper and lower proximal end-side first blood processing members (5/PEU, PED) and the pair of upper and lower distal end-side second blood processing members (5/DEU, DED),

the blood inlet section (7I) communicates with the proximal end-side first blood inflow chamber (2IR/PE), and

the blood outlet section (7O) communicates with the distal end-side second blood inflow chamber (2IR/DE).

[9] The present invention provides the blood processing device (101) described in [8],

wherein an outer edge of each of the pair of upper and lower proximal end-side first blood processing members (5/PEU, 5/PED) is fixed to an inner edge of a pair of upper and lower proximal end-side first outer frame sheets (3/PEU, 3/PED) in a state where the outer edge is held by the pair of upper and lower proximal end-side first outer frame sheets (3/PEU, 3/PED), and

an outer edge of the pair of upper and lower proximal end-side first outer frame sheets (3/PEU, 3/PED) is fixed to the housing (2), and

an outer edge of each of the pair of upper and lower distal end-side second blood processing members (5/DEU, 5/DED) is fixed to an inner edge of a pair of upper and lower distal end-side second outer frame sheets (3/DEU, 3/DED) in a state where the outer edge is held by the pair of upper and lower distal end-side second outer frame sheets (3/DEU, 3/DED), and

an outer edge of the pair of upper and lower distal end-side second outer frame sheets (3/DEU, 3/DED) is fixed to the housing (2).

[10] The present invention provides the blood processing device (101) described in [9],

wherein a pair of upper and lower first lengthened sections (3L/PEU, 3L/PED) are fixed to the proximal end (PE) side of the pair of upper and lower proximal end-side first outer frame sheets (3/PEU, 3/PED),

a pair of upper and lower second lengthened sections (3L/DEU, 3L/DED) are fixed to the distal end (DE) side of the pair of upper and lower distal end-side second outer frame sheets (3/DEU, 3/DED),

on the proximal end (PE) side where the blood inlet section (7I) is fixed, from the upper portion (U) side to the lower portion (D) side, the first housing (2/U), the first lengthened section (3L/PEU), the blood inlet section (7I), the first lengthened section (3L/PED), and the second housing (2/D) are fixed in this order, and

on the distal end (DE) side where the blood outlet section (7O) is fixed, from the upper portion (U) side to the lower portion (D) side, the first housing (2/U), the second lengthened section (3L/DEU), the blood outlet section (7O), the second lengthened section (3L/DED), and the second housing (2/D) are fixed in this order.

[11] The present invention provides the blood processing device (101) described in [8],

wherein an inlet side hole (8I) is formed at a position closer to the proximal end (PE) side than an inlet opening (7IO) on an extreme distal end (DE) side of the blood inlet section (7I), and

the inlet side hole (8I) is formed in the vicinity of the proximal end (PE) side of an inner side filtration section (5FI/PE) of the proximal end-side first blood processing member (5/PEU, 5/PED), and

an outlet side hole (8O) is formed at a position closer to the distal end (DE) side than an outlet opening (7OO) on an extreme proximal end (PE) side of the blood outlet section (7O), and

the outlet side hole (8O) is formed in the vicinity of the distal end (DE) side of an inner side filtration section (5FI/DE) of the distal end-side second blood processing member (5/DEU, 5/DED).

[12] The present invention provides a blood processing device (201), comprising:

an upper portion-side first housing (2/U) and a lower portion-side second housing (2/D) formed of a thick flexible sheet processed into a three-dimensional shape;

a blood inlet section (7I) disposed on a proximal end (PE) side of the upper portion-side first housing (2/U) and the lower portion-side second housing (2/D);

a blood outlet section (7O) disposed on a distal end (DE) side of the upper portion-side first housing (2/U) and the lower portion-side second housing (2/D); and

a blood processing member (5) which is disposed in the upper portion-side first housing (2/U) and the lower portion-side second housing (2/D) and formed by laminating a plurality of sheets of nonwoven cloth having a predetermined thickness,

wherein the blood processing member (5) has an inner side filtration section (5FI) and an outer side filtration section (5FO),

a blood inflow chamber (2IR) is formed between the inner periphery of the upper portion-side first housing (2/U) and the inner side filtration section (5FI) of the blood processing member (5),

a blood outflow chamber (2OR) is formed between the inner periphery of the lower portion-side second housing (2/D) and the outer side filtration section (5FO) of the blood processing member (5),
the blood inlet section (7I) communicates with the blood inflow chamber (2IR),
the blood outlet section (7O) communicates with the blood outflow chamber (2OR),
the first housing (2/U) and the second housing (2/D) are three-dimensionally formed such that an interior (2I) thereof rises from an outer edge portion (2F), and
a plurality of projections (T) are provided in the interior (2I) of the second housing (2/D) or in the interior (2I) of the second housing (2/D) and the first housing (2/U).

[13] The present invention provides the blood processing device (201) described in [12], wherein the first housing (2/U) and the second housing (2/D) are formed by processing a flexible sheet having a thickness equal to or greater than 0.8 mm and equal to or smaller than 1.5 mm into a three-dimensional shape, wherein
provided that a thickness of the first housing (2/U) is [d(U)] and a thickness of the second housing (2/D) is [d(D)], a ratio of [d(D)] to [d(U)] is 100:100 to 188:100.
[14] The present invention provides the blood processing device (201) described in [13], wherein the projections (T) have a height equal to or greater than 3 mm and equal to or smaller than 10 mm, and
an arrangement interval between the projections (T) is equal to or greater than 20 mm and equal to or smaller than 30 mm.
[15] The present invention provides the blood processing device (201) described in [12], wherein in the blood outlet section (7O), an outlet side hole (8O) is formed in the vicinity of a distal end-side sealing portion (2SDE).

Advantageous Effects of Invention

The present invention typically has embodiments 1 to 4. First, the characteristics of each of the embodiments and effects resulting from the characteristics will be specifically described.

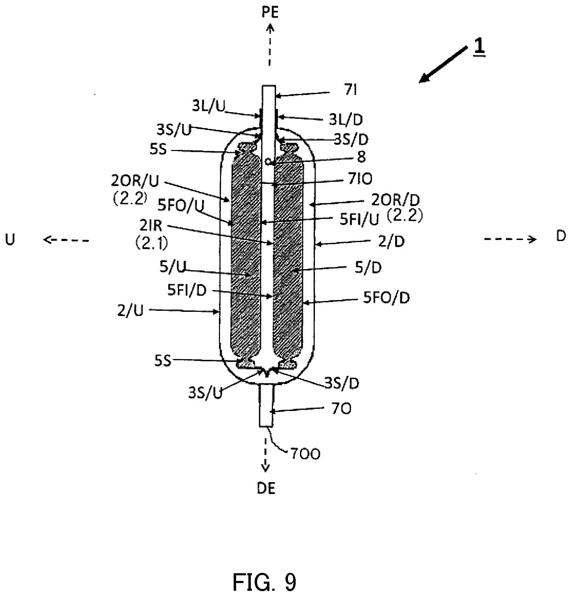

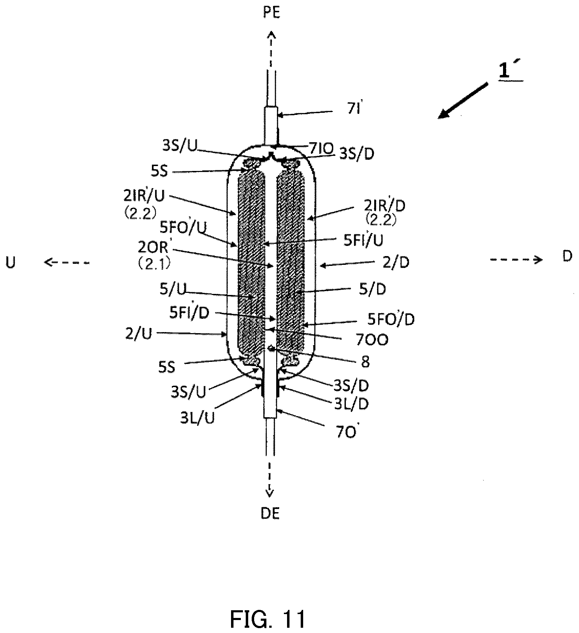

(1) First, blood processing devices (1 and 1') of the present invention (embodiments 1 and 2) will be described [FIG. 9 shows blood processing device 1 (hereinafter, referred to as "Type I" in some cases), and FIG. 11 shows blood processing device 1' (hereinafter, referred to as "Type II" in some cases)]. As described above in [1], in the interior of upper portion-side housing 2/U and lower portion-side housing 2D, a pair of upper and lower blood processing members

(5/U and 5/D) are disposed, and between inner side filtration sections (referred to as interior filtration sections as well) (5FI/U and 5FI/D), which face each other, of the pair of opposed upper and lower blood processing members (5/U and 5/D), inner side first blood chamber 2.1 is formed. Furthermore, between the inner periphery of housings (2/U and 2/D) and outer side filtration sections (5FO/U and 5FO/D) of the pair of blood processing members (5/U and 5/D), outer side second blood chamber 2.2 is formed.
In a case where blood inlet section 7I is formed to communicate with inner side first blood chamber 2.1, blood outlet section 7O is formed to communicate with outer side second blood chamber 2.2. Inner side first blood chamber 2.1 functions as blood inflow chamber 2IR, and outer side second blood chamber 2.2 functions as blood outflow chambers (2OR/U and 2OR/D).
Blood processing device 1 formed as above can filter blood in the interior of housings (2/U and 2/D), that is, by causing blood to move from inner side first blood chamber 2.1 (blood inflow chamber 2IR), pass through inner side filtration sections (5FI/U and 5FI/D) of upper portion-side first blood processing member 5/U and lower portion-side second blood processing member 5/D, and then pass through outer side filtration sections (5FO/U and 5FO/D). The filtered blood can be discharged through outer side second blood chamber 2.2 [blood outflow chambers (2OR/U and 2OR/D)].
Because blood processing device 1 is constituted as above, the internal pressure of outer side second blood chamber 2.2 [blood outflow chambers (2OR/U and 2OR/D)] becomes positive at the time of blood filtration. Furthermore, blood processing members (5/U and 5/D) can secure an effective filtration area that is substantially two times the effective filtration area of the inventions described in PTLS 1 to 4. Accordingly, the filtration time can be significantly reduced.
(2) In addition, blood processing device 1' ("Type II") can be constituted as below based on blood processing device 1 ("Type I"). That is, in blood processing device 1, proximal end PE and distal end DE are flipped 180°, blood inlet section 71' is formed to communicate with outer side second blood chamber 2.2, and blood outlet section 7O' is formed to communicate with inner side first blood chamber 2.1. By this constitution, outer side second blood chamber 2.2 can function as blood inflow chambers (2IR'/U and 2IR'/D), and inner side first blood chamber 2.1 can function as blood outflow chamber 2OR'. This is an embodiment of blood processing device 1' shown in FIG. 11.
Blood processing device 1' formed as above can filter blood by causing blood to move from outer side second blood chamber 2.2 [blood inflow chambers (2IR'/U and 2IR'/D)], pass through outer side filtration sections (5FO'/U and 5FO'/D) of upper portion-side first blood processing member 5/U and lower portion-side second blood processing member 5/D, and then pass through inner side filtration sections (5FI'/U and 5FI'/D). The filtered blood can be discharged through inner side first blood chamber 2.1 (blood outflow chamber 2OR').

Blood processing device 1' is constituted as above. Therefore, similarly to blood processing device 1, the internal pressure of outer side second blood chamber 2.2 [blood inflow chambers (2IR'/U and 2IR'/D)] becomes positive at the time of blood filtration, and blood processing members (5/U and 5/D) can secure an effective filtration area that is substantially two times the effective filtration area of the inventions described in PTLS 1 to 4. Therefore, the filtration time is expected to be significantly reduced.

(3) As described so far, blood processing devices (1 and 1') of the present invention are expected to greatly improve a leukocyte removal efficiency.

(4) Next, blood processing device 101 (hereinafter, referred to as "Type III" in some cases) of the present invention (embodiment 3) will be described. This device is obtained by subdividing the pair of upper portion-side and lower portion-side blood processing members (5/U and 5/D) of blood processing device 1 ("Type I") of the embodiment 1 into a pair of (two) upper and lower first blood processing members (5/PEU and PED) on the proximal end side and a pair of (two) upper and lower second blood processing members (5/DEU and DED) on the distal end side, such that a total of two pairs of (four) blood processing members 5 are disposed as described above in [8].

In [1] described above, in order to make sure that blood processing device 101 is also within the scope of a patent, a case where blood processing member 5 includes upper portion-side first blood processing members (5/PEU and 5/DEU) (in the PE-side area and the DE-side area) and lower portion-side second blood processing members (5/PED and 5/DED) (in the PE-side area and the DE-side area) is stipulated.

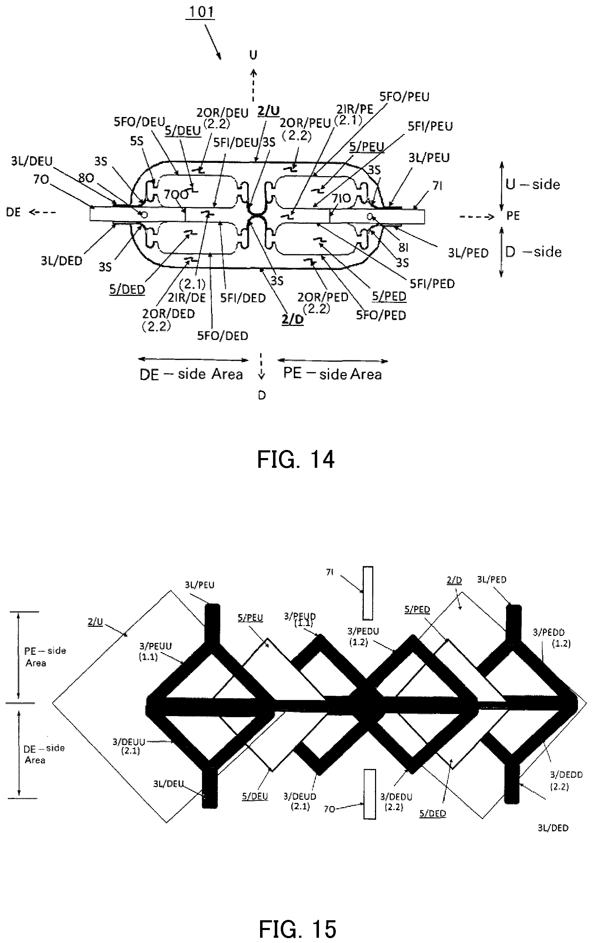

In blood processing device 101, blood can be filtered as described below (see FIG. 14 and the like).

That is, from blood inlet section 7I formed on the proximal end PE side, blood to be processed flows into proximal end-side (interior) first blood inflow chamber 2IR/PE, and is then filtered through proximal end-side first blood processing members (5/PEU and PED) on the proximal end PE side disposed on the upper portion U side and the lower portion D side of first blood inflow chamber 2IR/PE, toward the outside from the inside.

The filtered blood moves to second blood outflow chamber 2OR/DE on the distal end DE side from first blood outflow chamber 2OR/PE on the proximal end PE side, is filtered through distal end-side second blood processing members (5/DEU and DED) on the distal end DE side from second blood outflow chamber 2OR/DE toward the inside from the outside, and flows into distal end-side (interior) second blood inflow chamber 2IR/DE.

The filtered blood is discharged from second blood inflow chamber 2IR/DE through blood outlet section 7O.

In blood processing device 101 (Type III) of the present invention constituted as above (embodiment 3), the blood processing members (disposed in four areas) (5/PEU, PED, DEU, and DED) can secure an effective filtration area larger than that of blood processing devices (1 and 1').

That is, the blood processing members are prepared by subdividing the pair of upper and lower blood processing members (5/U and 5/D) of blood processing device 1 of embodiment 1, such that one pair is disposed in an area on the proximal end side and the other pair is disposed in an area of the distal end side (a total of four blood processing members are disposed). Therefore, it is reasonable to expect that blood processing device 101 can significantly further reduce the filtration time compared to the inventions in PTLS 1 to 4 and the blood processing devices (1 and 1') of the embodiments 1 and 2. As a result, a leukocyte removal efficiency is expected to be improved as well.

(5) In addition, blood processing device 201 (hereinafter, referred to as "Type IV" in some cases) of the present invention (embodiment 4) will be described. This device has upper portion-side first housing 2/U and lower portion-side second housing 2/D obtained by processing a thick flexible sheet into a three-dimensional shape. Upper portion-side first housing 2/U and lower portion-side second housing 2/D are three-dimensionally formed such that interior 2I rises from outer edge portion 2F. Interior 2I of lower portion-side second housing 2/D or interior 2I of second housing 2/D and upper portion-side first housing 2/U are provided with a plurality of projections T.

Blood processing device 201 of the present invention is constituted as above. Therefore, even though the internal pressure of blood outflow chamber 2OR becomes negative, the inner wall of lower portion-side second housing 2/D is deformed to a small extent in a direction along which the inner wall contacts the outer side filtration section 5OF side of blood processing member 5. Furthermore, even though deformation occurs in the direction along which the inner wall contacts the outer side filtration section 5OF side, due to projections T which are provided on the inner wall of lower portion-side second housing 2/D and protrude toward outer side filtration section 5OF side, the contact with outer side filtration section 5OF side is hindered.

Accordingly, the blood flow path in blood outflow chamber 2OR can be sufficiently secured. Furthermore, because the blood flow path in the vicinity of outlet opening 7OO of blood outlet section 7O is not blocked, the filtration time can be significantly reduced.

(6) Furthermore, in a case where side hole 8 is formed in the vicinity of distal end DE-side sealing portion 2SDE of blood outlet section 7O, it is possible to further accelerate the outflow of blood. Therefore, the filtration time can be further reduced.

Brief Description of Drawings

FIG. 1 is a general view (plan view/schematic view) showing an example (embodiment 1) of blood processing device 1 (Type I) of the present invention;

FIG. 2 is an exploded perspective view of blood processing device 1 in FIG. 1;

FIG. 3 is a schematic view showing a state where upper portion-side blood processing member 5/U of blood processing device 1 is fixed to outer frame sheet 3;

FIG. 4 is a schematic view showing a state where upper portion-side blood processing member 5/U and lower portion-side blood processing member 5/D are in the process of being fixed to each other;

FIG. 5 is a schematic view showing a state where blood processing member 5 is in the process of being fixed to flexible housing 2, the drawing schematically shows a state where corners CR/S1 and CR/S2 of each of first side portion S1 and second side portion S2 are in the process of being fixed as corner sealing portions SCR/S1 and SCR/S2;

FIG. 6 is a schematic view showing a state where blood inlet section 7I and blood outlet section 7O are in the process of being fixed to flexible housing 2 after the process shown in FIG. 5;

FIG. 7 is a schematic view showing a state where blood processing member 5 is in the process of being fixed to flexible housing 2 through outer frame sheet 3 after the process shown in FIG. 6;

FIG. 8 is a schematic view of blood processing device 1 completed by removing unnecessary flexible sheet SH from FIG. 7;

FIG. 9 is a cross-sectional view of blood processing device 1 in FIG. 1 taken along line A-A';

FIG. 10 is a view (cross-sectional view) showing a usage state of blood processing device 1 in FIG. 9;

FIG. 11 is a cross-sectional view showing an example (embodiment 2) of blood processing device 1' (Type II) of the present invention (corresponding to a cross-sectional view of blood processing device 1 in FIG. 1 taken along line A-A');

FIGS. 12A and 12B are views (cross-sectional views) showing a usage state of each of blood processing devices 1 and 1', FIG. 12A is a view (cross-sectional view) showing a usage state of blood processing device 1' (Type II), and FIG. 12B is a view (cross-sectional view) showing a usage state of blood processing device 1 (Type I);

FIG. 13 is a general view (plan view/schematic view) showing an example (embodiment 3) of blood processing device 101 (Type III) of the present invention;

FIG. 14 is a cross-sectional view of FIG. 13 taken along line A-A';

FIG. 15 is an exploded perspective view of FIG. 13;

FIG. 16 is a schematic view showing a state where upper portion-side blood processing member 5/U is fixed to outer frame sheet 3;

FIG. 17 is a schematic view showing a state where upper portion-side blood processing member 5/U and lower portion-side blood processing member 5/D are in the process of being fixed to each other;

FIG. 18 is a schematic view showing a state where blood processing member 5 is in the process of being fixed to flexible sheet SH, the drawing schematically shows a state where corners CR/S1 and CR/S2 of first side portion S1 and second side portion S2 are in the fixing process;

FIG. 19 is a schematic view showing a state where blood inlet section 7I and blood outlet section 7O are in the process of being fixed to flexible sheet SH after the process shown in FIG. 18;

FIG. 20 is a schematic view showing a state where blood processing member 5 is in the process of being fixed to flexible sheet SH through outer frame sheet 3 after the process shown in FIG. 19;

FIG. 21 is a schematic view of blood processing device 101 completed by removing unnecessary flexible sheet SH from FIG. 20;

FIG. 22 is a view showing a usage state of FIG. 20;

FIG. 23 is a general view (plan view/cross-sectional view) showing an example (embodiment 4) of blood processing device 201 (Type IV) of the present invention in a usage state;

FIG. 24 is a general view (bottom view/schematic view) showing an example of blood processing device 201 of the present invention;

FIG. 25 is a general view (bottom view/schematic view) showing an example of blood processing device 201 of the present invention;

FIG. 26 is a general view (perspective view/schematic view) showing an example of blood processing device 201 of the present invention;

FIG. 27 is a partially enlarged view of FIG. 26;

FIG. 28 is an exploded perspective view showing an example of blood processing device 201 of the present invention;

FIG. 29 is a cross-sectional view of FIG. 23 taken along line A-A';

FIG. 30 is a view (cross-sectional view) showing a usage state of blood processing device 201 in FIG. 29; and

FIG. 31 is a view (bottom view) showing a usage state of blood processing device 201 in FIG. 29.

Description of Embodiments

(Definition)

Hereinafter, the present invention will be specifically described with reference to drawings. For the convenience of description, in order to clearly explain blood processing devices 1 (Type I), 1' (Type II), 101 (Type III), and 201 (Type IV) of embodiments 1 to 4 of the present invention, the following definitions are given based on the disposition shown in the drawings.

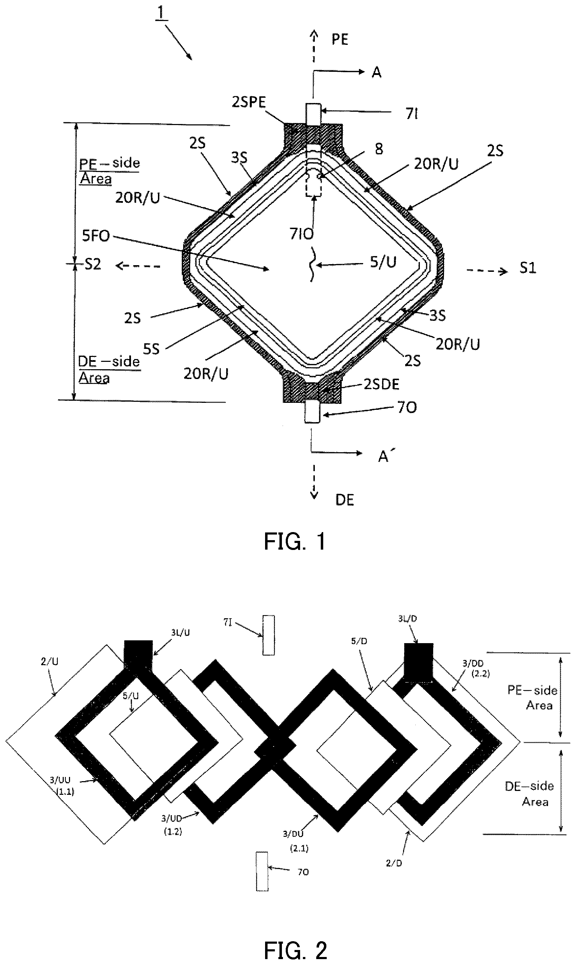

The proximal end PE side (referred to as direction as well, the same shall be applied to the following description) means blood inlet section 7I side as shown in FIG. 1, for example.

The distal end DE side means the blood outlet section 7O side as shown in FIG. 1, for example.

The first side portion S1 side means a right direction on a line connecting blood inlet section 7I and blood outlet section 7O in series as shown in FIG. 1, for example.

The second side portion S2 side means a direction opposite to the first side portion S1 side as shown in FIG. 1, for example. That is, the second side portion S2 side means a left direction on a line connecting blood inlet section 7I and blood outlet section 7O in series.

The upper portion U side means the upper side in the paper. In FIG. 1, the upper portion U side means the direction of the front side of the paper.

The lower portion D side means the lower side in the paper. In FIG. 1, the lower portion D side means the direction of the rear side of the paper.

For the combinations of the proximal end PE side and the distal end DE side; first side portion S1 and second side portion S2; and upper portion U and lower portion D, each of the elements of the combinations is referred to as "one direction of a side portion" in some cases. For example, provided that the proximal end-PE side is referred to as "one direction of a side portion", the distal end DE side opposite to the proximal end PE side is referred to as "one direction of the other side portion" in some cases. The same is true for the case of first side portion S1 and second side portion S2 and upper portion U and lower portion D.

The line connecting blood inlet section 7I and blood outlet section 7O in series is referred to as approximate center in a longitudinal direction in some cases.

In some cases, the direction of first side portion S1, upper portion U, second side portion S2, and lower portion D is referred to as outer peripheral direction in the longitudinal direction relative to the approximate center in the longitudinal direction.

As shown in FIGS. 1 to 10, in a case where a pair of members such as blood processing members 5 (generally, constituted with, for example, a plurality of sheets of laminated nonwoven cloth as will be described later) are present in upper portion U and lower portion D in blood processing device 1, the members are described as below. For example, for the upper portion U side, just like blood processing member 5/U, "U" is added after the reference sign "5" such that the member is marked with "5/U" meaning blood processing member 5/U on the upper portion U side (similarly, by being marked with "5/D", the member represents blood processing member 5/D on the lower portion D side).

More examples will be given regarding the notation method described above. For example, just like outer frame sheet 3, in a case where a pair of members (outer frame sheet 3) for holding processing member 5 of upper portion U are present in upper portion U, and a pair of members (outer frame sheet 3) for blood processing member 5 of lower portion D are present in lower portion D, for instance, the pair of members (outer frame sheet 3) on the upper portion U side are described as "3/UU" and "3/UD", and the pair of members (outer frame sheet 3) in the lower portion D side are described as "3/DU" and "3/DD" (particularly, see FIG. 2).

Blood processing member 5/U on the upper portion U side is described as upper portion-side first blood processing member 5/U in some cases, and blood processing member 5/D on the lower portion D side is described as lower portion-side second blood processing member 5/D in some cases. Other members on the upper portion U side are described as "upper portion-side first ··· /U" in some cases. Similarly, members on the lower portion D side are described as "lower portion-side second ··· /D" in some cases.

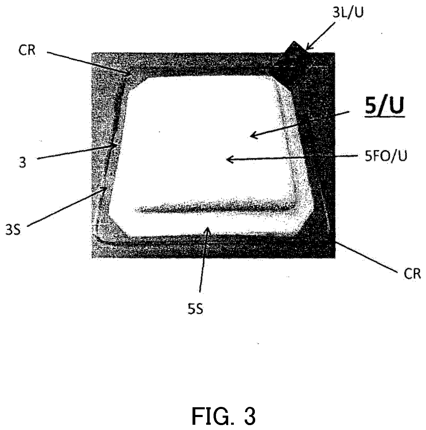

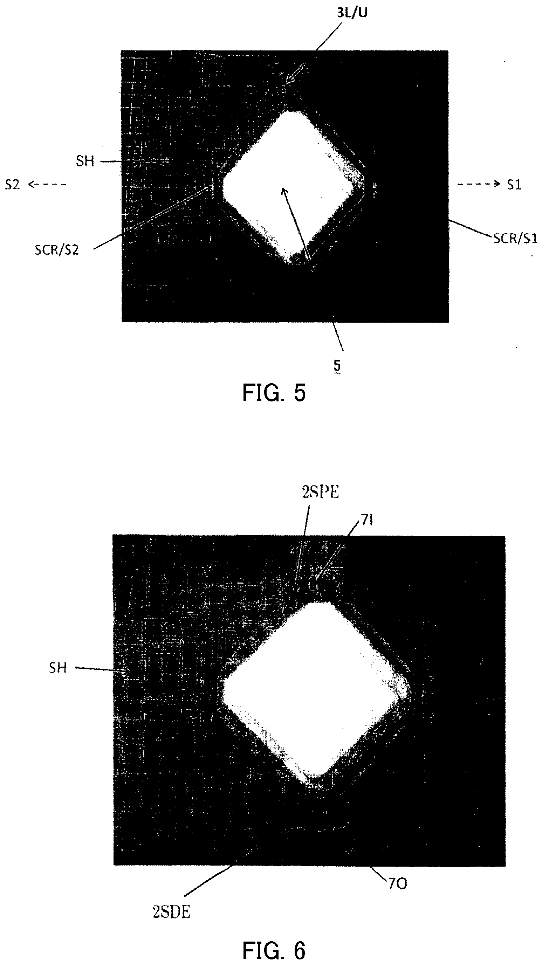

For example, in a case where there is a pair of members just like blood processing members 5, and these are shown in a drawing, two kinds of reference signs such as "5/U and 5/D" are used. In a case where the members are not shown in a drawing such as FIG. 3 or FIG. 5, in order to avoid making reference signs in the drawing complicated, only one reference sign such as "5" is used. Here, in the description of the invention and the like, two kinds of reference signs such as "5/U and 5/D" are used in some cases.

For instance, in a case where a certain member consists of a pair of (two) members just like a pair of (two) blood processing member 5/U on the upper portion U side and blood processing member 5/D on the lower portion D side, sometimes the reference signs thereof are collectively used just like blood processing members (5/U and 5/D) or (5/U and D).

Furthermore, most of the members of blood processing device 1 (Type I) and blood processing device 1' (Type II) have the same form (shape and structure) and are constituted with the same material. The devices are different from each other in terms of the usage method, the name of each of the members changing according to the usage method, and the like. Therefore, only the different sections will be mainly described.

For example, in some cases, for the convenience of description, blood inflow chambers 2IR of blood processing device 1 will be collectively called first blood chamber 2.1, and blood outflow chambers (2OR/U and 2OR/D) will be collectively called second blood chamber 2.2.

In addition, in some cases, blood inflow chambers (2IR'/U and 2IR'/D) of blood processing device 1' will be collectively called second blood chamber 2.2, and blood outflow chambers 2OR' will be collectively called first blood chamber 2.1.

(Reference signs of members and relationship between upper concept and lower concept of reference signs)

The reference signs of the respective members are used merely for facilitating understanding of the shape, the position (disposition), and the like of the respective members in exemplary embodiments of the present invention. Therefore, the scope of a patent of the present invention is not limited to the shape and the position (disposition) represented by the reference signs.

(Housing 2)

"Housing 2" means the uppermost concept of a housing (hereinafter, in order to avoid lengthy explanation, only reference signs will be used for description as far as possible).

For example, in a case where a suffix such as "/U" is written after a reference sign "2" just like the housing marked with a reference sign "2/U" or the like, the housing marked with the reference sign "2/U" or the like means a concept lower than "housing 2". Therefore, in a case where a housing is simply described as "housing 2" in the description of the invention and claims, the term means that the housing 2 includes or sometimes includes both the housings marked with reference signs "2/U" and "2/D" of the lower concept.

(Blood processing member 5)

"Blood processing member 5" means the uppermost concept of a blood processing member. For example, in a case where "/U" (suffix specifying the area, the position, or the like where blood processing member 5 is disposed) or the like is written after a reference sign "5" just like the blood processing member marked with a reference sign "5/U" or the like, the blood processing member marked with the reference sign "5/U" or the like means a (more specifically extended) concept lower than "blood processing member 5".

The relationship between the blood processing members marked with reference signs "5", "5/PE", and "5/PEU" is the same as described above.

Accordingly, in a case where a blood processing member is simply described as blood processing member 5 in the description of the invention and claims, the term means that the blood processing member 5 includes or sometimes includes the blood processing members marked with reference signs "5/U" and "5/D" or "5/PE" or "5/DE" of a lower concept and the blood processing members marked with "5/PEU", "5/PED", "5/DEU", and "5/DED" of a much lower concept. Furthermore, in a case where a blood processing member is described as "blood processing member 5/PE", this member includes or sometimes includes blood processing members marked with reference signs "5/PEU" and "5/PED".

(Outer frame sheet 3)

"Outer frame sheet 3" means the uppermost concept of an outer frame sheet. For example, in a case where a suffix "/U" or the like is written after a reference sign "3" just like an outer frame sheet marked with a reference sign "3/U" or the like, the outer frame sheet marked with the reference sign "3/U" or the like means a concept lower than "outer frame sheet 3".

Furthermore, as in an outer frame sheet marked with a reference sign "3/UU", in a case where "U" or the like is written after a reference sign "3/U" or the like, the outer frame sheet marked with the reference sign "3/UU" means a concept lower than the outer frame sheets marked with the reference sign "3" and the reference sign "3/U".

Accordingly, in a case where an outer frame sheet is simply described as outer frame sheet 3 in the description of the present invention and claims, the term means that the outer frame sheet 3 includes or sometimes includes outer frame sheets marked with a reference sign "3/U" and a reference sign "3/D" and outer frame sheets marked with reference signs "3/UU", "3/UD", "3/DU", and "3/DD" of a much lower concept.

The relationship between outer frame sheets marked with reference signs "3", "3/PE", "3/PEU", and "3/PEUU" is the same as described above.

(Other members)

The relationship between an upper concept and a lower concept described above is also applied to other members, blood inflow chamber 2IR, blood outflow chamber 2OR, housing sealing portions 2S and 3S, outer frame sealing portion 3S, lengthened section 3L, filtration section 5F, inner side filtration section FI, outer side filtration section FO, and the like. Therefore, detailed descriptions thereof will not be repeated.

(Blood processing device 1 (Type I) of embodiment 1) (see FIGS. 1 to 10)

As shown in FIG. 1 to FIG. 10, blood processing device 1 (Type I) of the embodiment 1 of the present invention has flexible housing 2 (hereinafter, simply described as housing 2) formed of a flexible sheet and blood processing members 5 installed in the interior of housing 2.

Housing 2 includes housing 2/U on the upper portion U side and housing 2/D on the lower portion D side, and is in the form of a bag (referred to as bag-like container as well) obtained by bonding the peripheries of 2/U and 2/D.

On the proximal end PE side of housing 2, tubular (or pipe-like) blood inlet section 7I is mounted on one end portion of the approximate center. Meanwhile, on the distal end DE side of housing 2, tubular blood outlet section 7O is mounted on one end portion of the approximate center (for example, see FIG. 1).

It is preferable that tubular blood inlet section 7I and blood outlet section 7O are formed of a resin harder than flexible housing 2 formed of a flexible sheet such that 7I and 7O can be suitably connected to a tubular blood circuit.

As shown in FIG. 1, blood inlet section 7I includes main inlet opening 710 and auxiliary side hole 8 formed on the distal end DE side.

One of the characteristics of blood processing member 5 is that it makes a pair, that is, a pair of upper and lower members constituted with blood processing member 5/U on the upper portion U side and blood processing member 5/D on the lower portion D side.

Specifically, each of upper portion-side blood processing member 5/U and lower portion-side blood processing member 5/D is a blood processing filter member formed of a plurality of sheets of laminated nonwoven cloth.

(Assembly process of blood processing device 1)

Hereinafter, an aspect of an example of blood processing device 1 will be described together with an example of an assembly process.

As illustrated in FIG. 3, to be brief, blood processing device 1 is obtained by fixing blood processing member 5 by using outer frame sheet 3.

More specifically, as the most basic disposition, blood processing member 5 is constituted with a pair of upper and lower blood processing members. The outer edge of upper portion-side blood processing member 5/U is fixed to the inner edges of a pair of upper (first of the first) outer frame sheet 3/UU and lower (second of the first) outer frame sheet 3/UD on the upper portion U side in a state of being held between the inner edges of 3/UU and 3/UD (see FIG. 2).

In contrast, the outer edge of lower portion-side blood processing member 5/D is fixed to the inner edges of a pair of upper (first of the second) outer frame sheet 3/DU and lower (second of the second) outer frame sheet 3/DD on the lower portion D side in a state of being held between the inner edges of 3/DU and 3/DD (see FIG. 2).

Fixing of 5/U and 5/D can be performed by welding by means of external heating such as heat sealing or performed by welding by means of internal heating or the like using a high frequency or an ultrasonic wave. Furthermore, 5/U and 5/D may be fixed using an adhesive.

Hereinafter, the fixing portion of blood processing member 5 and outer frame sheet 3 will be referred to as "sealing portion 5S" in some cases. Sites other than the fixing portion are regarded as "non-fixing portion". Specifically, the non-fixing portion on the inside of blood processing member 5 and outer frame sheet 3 forms a site referred to as filtration section 5F. Regarding filtration section 5F, a surface on the blood inflow chamber 2IR side will be referred to as inner side filtration section 5FI in some cases, and a surface on the blood outflow chamber 2OR side will be referred to as outer side filtration section 5FO in some cases.

The outer edges of the pair of upper (first of the first) outer frame sheet 3/UU and lower (second of the first) outer frame sheet 3/UD on the upper portion U side are fixed to each other. Furthermore, the outer edges of the pair of upper (first of the second) outer frame sheet 3/DU and lower (second of the second) outer frame sheet 3/DD on the lower portion D side are fixed to each other.

Hereinafter, these fixing portions will be referred to as "outer frame sealing portion 3S (3S/U and 3S/D) in some cases.

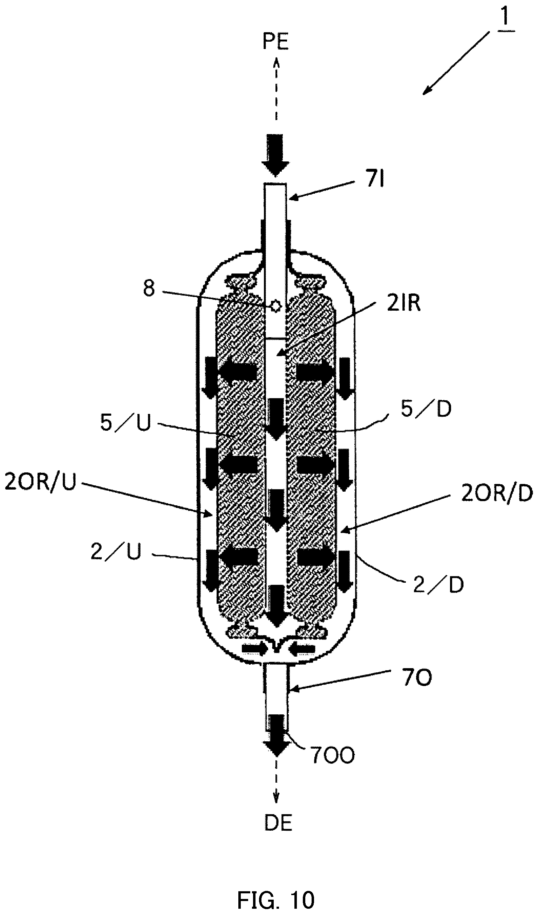

Upper portion-side lengthened section 3L/U is formed on the proximal end PE side of upper (first of the first) outer frame sheet 3/UU on the upper portion U side, and lower portion-side lengthened section 3L/D is formed on the proximal end PE side of lower (second of the second) outer frame sheet 3/DD on the lower portion D side (see FIG. 2). Tubular blood inlet section 71 is inserted·fixed between upper portion-side lengthened section 3L/U and lower portion-side lengthened section 3L/D (FIGS. 9 and 10).

As illustrated in FIG. 3, blood processing member 5 is compressed further in the fixing portion (sealing portion 5S) of blood processing member 5 and outer frame sheet 3 than in the non-fixing portion (filtration section 5F) on the inside of sealing portion 5S.

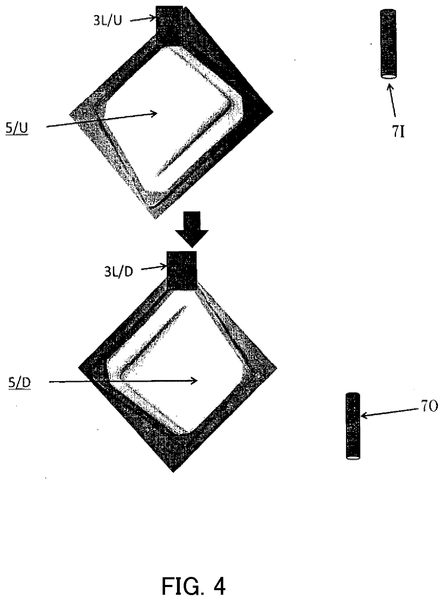

Then, as illustrated in FIG. 4, upper portion-side blood processing member 5/U and lower portion-side blood processing member 5/D are laminated such that inner side filtration sections 5FI/U and 5FI/D of 5/U and 5/D face each other. On the proximal end PE side between 5/U and 5/D, upper portion-side lengthened section 3L/U, tubular blood inlet section 7I, and lower portion-side lengthened section 3L/D are disposed, and on the distal end DE side, tubular blood outlet section 7O is disposed.

Thereafter, as illustrated in FIG. 5, the pair of blood processing member 5/U on the upper portion U side and blood processing member 5/D on the lower portion D side are covered with a pair of sheets, that is, a pair of upper and lower sheets SH/U and SH/D (becoming housing 2/U on the upper portion U side and housing 2/D on the lower portion D side). Subsequently, corners CR/S1 and CR/S2 on first side portion S1 side and second side portion S2 side are fixed by heat sealing (hereinafter, the fixing portions will be referred to as "corner sealing portion SCR" in some cases).

Then, as illustrated in FIG. 6, upper portion-side lengthened section 3L/U, blood inlet section 7I, lower portion-side lengthened section 3L/D, blood outlet section 7O, and the peripheries (edge portions or vicinities) of these are fixed to the pair of upper and lower sheets SH/U and SH/D by heat sealing or the like.

Hereinafter, the fixing portions will be referred to as "proximal end-side sealing portion 2SPE and distal end-side sealing portion 2SDE" in some cases.

The above portions may be fixed in any order. For example, blood outlet section 7O may be first fixed to sheets SH/U and SH/D by heat sealing or the like, and then blood inlet section 7I and lengthened sections 3L/U and 3L/D may be fixed. Alternatively, blood inlet section 7I and lengthened sections 3L/U and 3L/D may be fixed first, and then blood outlet section 7O may be fixed.

As another option, blood inlet section 7I, lengthened sections 3L/U and 3L/D, and blood outlet section 7O may be fixed simultaneously.

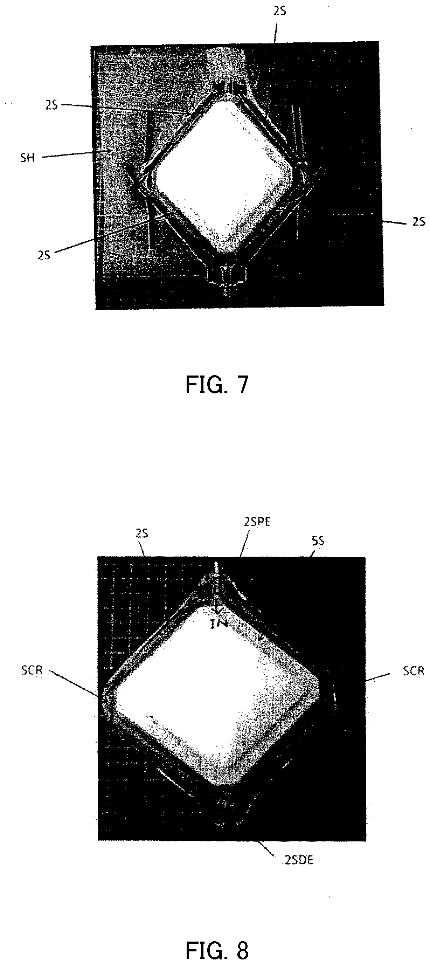

Thereafter, as illustrated in FIG. 7, outer frame sealing portions 3S/U and 3S/D of the outer edges of 4 sides (oblique sides) of blood processing member 5 are fixed to sheet SH/U and sheet SH/D by heat sealing or the like. Hereinafter, the fixing portions will be referred to as "sealing portion 2S" in some cases. In this way, 4 peripheries of blood processing member 5 are sealed by 4 sealing portions 2S. That is, a sealing unit including blood inlet section 7I on the proximal end side and blood outlet section 7O on the distal end side is formed.

Last, unnecessary sheets SH/U and SH/D of the outer edge are removed (cut) from each of sealing portions 2S and SCR, thereby completing blood processing device 1 in FIG. 8.

In order to easily remove (cut) SH/U and SH/D from each of sealing portions 2S and SCR, the tip of a heat sealing electrode or the like may be made approximately triangular such that the tip welding portion becomes thick after heat sealing. Alternatively, by causing a cutter to be adjacent to the heat sealing electrode or the like, a thick portion (cut line) can be formed in each of sealing portions 2S and SCR.

(Form of blood processing device 1)

The form of blood processing device 1 will be described.

For example, as shown in FIG. 9, on the proximal end PE side, at the site on which blood inlet section 7I is mounted, from the upper portion U side to the lower portion D side, upper portion-side housing 2/U, upper portion-side lengthened section 3L/U, blood inlet section 7I, lower portion-side lengthened section 3L/D, and lower portion-side housing 2/D are fixed in this order.

The proximal end PE side of upper portion-side blood processing member 5/U is fixed to upper portion-side lengthened section 3L/U through outer frame sealing portion 3S/U of a pair of upper and lower outer frame sheets 3/UU and 3/UD on the upper portion U side.

The proximal end PE side of lower portion-side blood processing member 5/D is fixed to lower portion-side lengthened section 3L/D through outer frame sealing portion 3S/D of a pair of upper and lower outer frame sheets 3/DU and 3/DD on the lower portion D side.

In contrast, on the distal end DE side, at the site on which blood outlet section 7O is mounted, from the upper portion U side to the lower portion D side, upper portion-side housing 2/U, blood outlet section 7O, and lower portion-side housing 2/D are fixed in this order.

The distal end DE side of upper portion-side blood processing member 5/U and lower portion-side Blood processing member 5/D is fixed through outer frame sealing portion 3S/U of a pair of upper and lower outer frame sheets 3/UU and 3/UD on the upper portion U side and outer frame sealing portion 3S/D of a pair of upper and lower outer frame sheets 3/DU and 3/DD on the lower portion D side.

In housing 2, upper portion-side inner side filtration section 5FI/U and lower portion-side inner side filtration section 5FI/D are disposed to face each other, and as a result, blood inflow chamber 2IR is formed. The distal end DE side of blood inlet section 7I is stuck into the interior of blood inflow chamber 2IR of housing 2 (FIG. 9). Side hole 8 is formed at a position closer to the proximal end PE side than to inlet opening 7IO on the extreme distal end DE side of blood inlet section 7I.

Side hole 8 is formed in the vicinity of proximal end PE side of inner side filtration sections 5FI/U and 5FI/D of blood processing members 5/U and 5/D in blood inflow chamber 2IR. Side hole 8 may be formed at 2 sites or formed at 3 to 4 sites.

In a case where blood is caused to flow into housing 2 only through inlet opening 7IO on the lower side of tubular blood inlet section 7I, it is apprehended that the distal end DE side of blood inlet section 7I may be crushed by being compressed by upper portion-side blood processing member 5/U and lower portion-side blood processing member 5/D, and a blood flow path could not be secured. However, in a case where side hole 8 is additionally formed in side portions, even though the distal end DE side of blood inlet section 7I is crushed and blocked, it is possible to make an excess of blood stably flow into housing 2 from side hole 8.

(Internal structure of housing 2)

The internal structure of housing 2 will be specifically described.

As shown in FIG. 9, between the lower portion D side of upper portion-side blood processing member 5/U and the upper portion U side of lower portion-side blood processing member 5/D, at the approximate center of housing 2 in the longitudinal direction, that is, on a line (area) directly connecting the distal end DE side of blood inlet section 7I and the proximal end PE side of blood outlet section 7O in series, a space is formed. This space functions as blood inflow chamber 2IR.

In contrast, as shown in FIG. 9, between the inner periphery of upper portion-side housing 2/U and lower portion D-side housing 2/D and the outer periphery (outer side filtration sections 5FO/U and 5FO/D) of upper portion-side blood processing member 5/U and lower portion-side blood processing member 5/D, spaces are formed. These spaces function as blood outflow chambers 2OR/U and 2OR/D. That is, the space closer to the distal end DE side than side hole 8 substantially functions as blood outflow chambers 2OR/U and 2OR/D.

Blood inlet section 7I communicates with blood inflow chamber 2IR, and blood outlet section 7O communicates with blood outflow chambers 2OR/U and 2OR/D.

(Flow of blood in blood processing device 1)

Next, the flow of blood in blood processing device 1 will be described.

As illustrated in FIG. 10, blood from the proximal end PE side of blood inlet section 7I passes through inlet opening 7IO on the distal end DE side and side hole 8 and flows into blood inflow chamber 2IR.

The blood in the blood inflow chamber 2IR passes through inner side filtration sections 5FI/U and 5FI/D of upper portion-side blood processing member 5/U and lower portion-side blood processing member 5/D, then passes through outer side filtration sections 5FO/U and 5FO/D, and moves into blood outflow chamber 2OR/U and 2OR/D. The internal pressure of blood outflow chambers 2OR/U and 2OR/D becomes positive. Therefore, the blood is rapidly discharged to the outside through blood outlet section 7O.

The blood in blood inflow chamber 2IR formed approximately at the approximate center of blood processing device 1 in the longitudinal direction can pass through the upper portion U side and the lower portion D side of blood inflow chamber 2IR, and can be filtered through the upper portion-side blood processing member 5/U and the lower portion-side blood processing member 5/D disposed on the outer periphery. Accordingly, blood processing device 1 can secure an effective filtration area that is substantially two times the effective filtration area of the blood processing devices disclosed in PTLS 1 to 4. Therefore, blood processing device 1 can significantly reduce the filtration time, and as a result, a leukocyte removal efficiency is expected to be improved as well.

(Function of side hole 8)

The significance of providing side hole 8 will be described again just in case. That is, in a case where blood is caused to flow into the blood processing device only through inlet opening 7IO of blood inlet section 7I, it is apprehended that the distal end DE side of blood inlet section 7I may be crushed by being compressed by upper portion-side blood processing member 5/U and lower portion-side blood processing member 5/D, and a blood flow path could not be secured. However, in a case where side hole 8 is provided, even though the distal end DE side of blood inlet section 7I is crushed, it is possible to make blood stably flow into the blood processing member from side hole 8.

As will be specifically described below, blood processing device 1 of the present invention can be used as blood processing device 1' (hereinafter, referred to as Type II) in FIG. 11 by flipping 180° proximal end PE and distal end DE in FIG. 1 (FIGS. 9 and 10) (hereinafter, referred to as Type I). In this case, the flow of blood in Type I is totally opposite to that in Type II.

That is, in Type II, blood outlet section 7O in Type I functions as blood inlet section 7I', and blood inlet section 7I in Type I functions as blood outlet section 7O'. Furthermore, in Type II, blood outflow chambers 2OR/U and 2OR/D (second blood chamber 2.2) in Type I function as blood inflow chambers 2IR'/U and 2IR'/D, and blood inflow chamber 2IR (first blood chamber 2.1) in Type I functions as blood outflow chamber 2OR'.

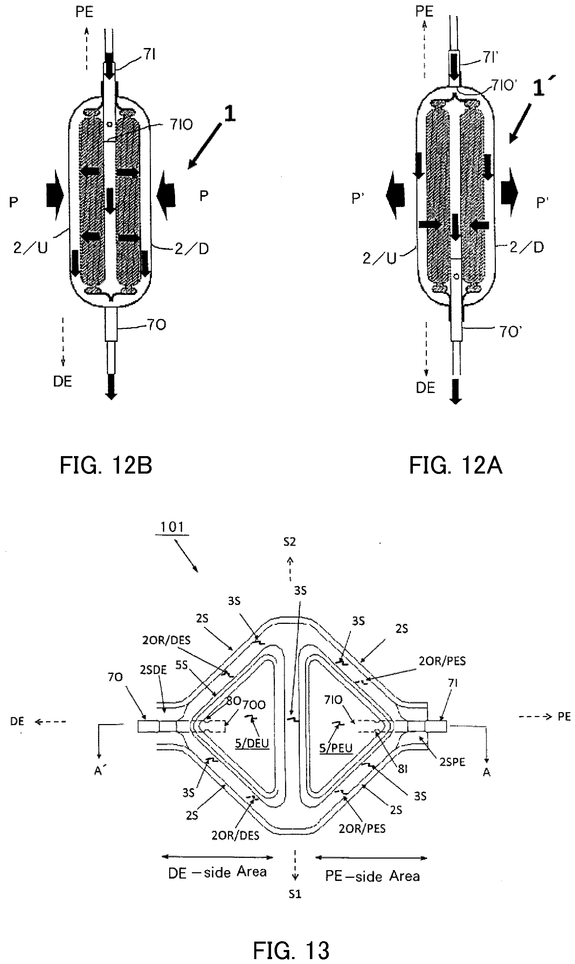

(Blood processing device 1' (Type II) of embodiment 2) (see FIGS. 11 and 12)

Blood processing device 1' (Type II) of embodiment 2 will be described. For example, as shown in FIG. 11, on the proximal end PE side, at the site on which blood inlet section 7I' is mounted, from the upper portion U side to the lower portion D side, upper portion-side housing 2/U, blood inlet section 7I', and lower portion-side housing 2/D are fixed in this order.

The proximal end PE side of upper portion-side blood processing member 5/U is fixed through outer frame sealing portion 3S/U of a pair of upper and lower outer frame sheets 3/UU and 3/UD on the upper portion U side.

The proximal end PE side of lower portion-side blood processing member 5/D is fixed through outer frame sealing portion 3S/D of a pair of upper and lower outer frame sheets 3/DU and 3/DD on the lower portion D side.

On the distal end DE side, at the site on which blood outlet section 7O' is mounted, from the upper portion U side to the lower portion D side, upper portion-side housing 2/U, upper portion-side lengthened section 3L/U, blood outlet section 7O', lower portion-side lengthened section 3L/D, and lower portion-side housing 2/D are fixed in this order.

The distal end DE side of upper portion-side blood processing member 5/U is fixed to upper portion-side lengthened section 3L/U through outer frame sealing portion 3S/U of a pair of upper and lower outer frame sheets 3/UU and 3/UD on the upper portion U side.

The distal end DE side of lower portion-side blood processing member 5/D is fixed to lower portion-side lengthened section 3L/D through outer frame sealing portion 3S/D of a pair of upper and lower outer frame sheets 3/DU and 3/DD on the lower portion D side.

(Function of side hole 8)

Similarly to the case of Type I, the proximal end PE side is stuck into the interior of blood outflow chamber 2OR' formed between upper portion-side blood processing member 5/U and lower portion-side blood processing member 5/D of housing 2. Side hole 8 is formed at a position closer to the distal end DE side than outlet opening 7OO on the extreme proximal end PE side of blood outlet section 7O' in the same manner as in Type I.

Side hole 8 is formed in the vicinity of distal end DE side of inner side filtration sections 5FI'/U and 5FI'/D of blood processing members 5/U and 5/D in blood outflow chamber 2OR'. Side hole 8 maybe formed at 2 sites or formed at 3 to 4 sites.

In a case where blood is caused to flow out of the blood processing device only through outlet opening 7OO of blood outlet section 7O', it is apprehended that the proximal end PE side of blood outlet section 7O' may be crushed by being compressed by upper portion-side blood processing member 5/U and lower portion-side blood processing member 5/D, and a blood flow path could not be secured. However, in a case where side hole 8 is formed, even though the proximal end PE side of blood outlet section 7O' is crushed to be blocked, it is possible to make blood stably flow out of side hole 8.

(Internal structure of housing 2)

The internal structure of housing 2 will be specifically described with reference to drawings.

Between the inner periphery of upper portion-side housing 2/U and lower portion D-side housing 2/D and the outer periphery (outer side filtration sections 5FO'/U and 5FO'/D) of upper portion-side blood processing member 5/U and lower portion-side blood processing member 5/D, spaces are formed. These spaces become upper and lower blood inflow chambers 2IR'//U and 2IR'/D.

Between the lower portion D side of upper portion U-side blood processing member 5/U and the upper portion U side of lower portion-side blood processing member 5/D, at the approximate center of housing 2 in the longitudinal direction, that is, on a line (area) connecting the distal end DE side of blood inlet section 7I' and the proximal end PE side of blood outlet section 7O' in series, a space is formed. This space functions as blood outflow chamber 2OR' (a portion closer to the proximal end PE side than side hole 8 substantially functions as blood outflow chamber 2OR').

Blood inlet section 7I' communicates with upper and lower blood inflow chambers 2IR'/U and 2IR'/D, and blood outlet section 7O' communicates with blood outflow chamber 2OR'.

(Flow of blood)

Next, the flow of blood in blood processing device 1' (Type II) will be described.

As illustrated in FIG. 12A (see the reference signs in FIG. 11 as well), the blood flowing into blood processing device 1' passes through opening 710' on the distal end DE side from the proximal end PE side of blood inlet section 7I', collides with outer frame sealing portions 3S/U and 3S/D on the distal end side, circles around the proximal end PE side of blood processing members 5/U and 5/D along the directions of upper portion U, lower portion D, and side portion S, and flows into upper portion U-side blood inflow chamber 2IR'/U and lower portion D-side blood inflow chamber 2IR'/D (in Type II, blood inflow chamber 2IR' is present as 2IR'/U and 2IR'/D at two sites on upper portion side U and lower portion side D (in the case of Type I, one blood inflow chamber 2IR is present in the central portion)).

The blood in blood inflow chamber 2IR' passes through outer side filtration sections 5FO'/U and 5FO'/D of upper portion-side blood processing member 5/U and lower portion-side blood processing member 5/D, then passes through inner side filtration sections 5FI'/U and 5FI'/D, and moves into blood outflow chamber 2OR'. The blood is then rapidly discharged to the outside through blood outlet section 7O'. After the blood is discharged, the internal pressure of blood outflow chamber 2OR' becomes negative.

Meanwhile, as illustrated in FIG. 12B (see the reference signs in FIG. 9 as well), head pressure of the blood flowing into the blood processing device 1 (Type I) from blood inlet section 7I on the proximal end PE side is concentrated on the approximate center of internal blood inflow chamber 2IR which is between and surrounded by blood processing member 5/U on the upper portion U side and blood processing member 5/D on the lower portion D side. The internal pressure of blood outflow chamber 2OR remains positive from when the blood filtration is started. However, as the blood filtration nears an end, after the blood is discharged to the outside from blood outflow chamber 2OR through blood outlet 7O, the internal pressure of blood outflow chamber 2OR (2OR/U and 2OR/D) becomes negative. It was confirmed that as a result, upper portion U-side housing 2/U adheres to outer side filtration section 5FO/U on the upper portion U side of blood processing member 5/U on the upper portion U side, lower portion D-side housing 2/D adheres to outer side filtration section 5FO/D on the lower portion D side of blood processing member 5/U on the lower portion D side, and hence the filtration speed tends to be reduced as the filtration nears an end.

As illustrated in FIG. 12A (see the reference signs in FIG. 11 as well), in blood processing device 1' (Type II), due to the inflow pressure (head pressure) of the blood flowing into the device from blood inlet section 7I' on the proximal end PE side, the internal pressure of blood inflow chambers 2IR'/U and 2IR'/D at two sites on the outside becomes positive. Furthermore, due to the inflow pressure (head pressure), housings 2/U and 2/D expand toward the outside/in lateral directions (direction of upper portion U and direction of lower portion D).

After the blood filtration, negative pressure is generated in blood processing device 1'. However, the negative pressure is generated only in one blood outflow chamber 2OR' at the center that is between and surrounded by blood processing member 5/U on the upper portion U side and blood processing member 5/D on the lower portion D side. Furthermore, blood outlet section 7O' (referred to as long port for so-called blood drainage) stuck deep into the interior of blood outflow chamber 2OR' plays a role of preventing crushing of the internal space (blood outflow chamber 2OR') of blood processing members 5/U and 5/D.

Therefore, while the blood filtration is being performed, the internal pressure of inner side filtration sections 5FI'/U and 5FI'/D of blood processing members 5/U and 5/D stays negative all the time. Accordingly, the blood filtration can be performed without reducing the filtration speed all the time.

(Constituent material of blood processing member 5)

As the constituent material of blood processing member 5, basically, it is preferable to use nonwoven cloth as described above.

Materials of the nonwoven cloth are not particularly limited, and for example, polyester such as polyethylene terephthalate, polytrimethylene phthalate, or polybutylene phthalate; polyamide such as nylon, nylon 6, nylon 11, nylon 12, or nylon 66; polyolefin such as polyethylene or polypropylene; polyurethane; polyvinyl chloride; acrylonitrile; styrene-based elastomer; and the like are used.

(Examples of size and constitution of nonwoven cloth)

A plurality of sheets of nonwoven cloth having the same fiber diameter may be laminated so as to obtain blood processing member 5 having a predetermined thickness. Alternatively, in order to more efficiently process blood, a plurality of sheets of nonwoven cloth having different fiber diameters may be laminated to obtain blood processing member 5 having a predetermined thickness.

The later constitution, in which a plurality of sheets of nonwoven cloth having different fiber diameters are combined, includes, for example, as described in Japanese Patent No. 3710384 (claims and paragraph [0015]), a constitution including three kinds of filters of (A) prefilter having average fiber diameter D equal to or greater than 5.0 $\mu$m and equal to or smaller than 10.0 $\mu$m, (B) first main filter having average fiber diameter D greater than 1.0 $\mu$m and equal to or smaller than 5.0 $\mu$m, and (C) second main filter having average fiber diameter D equal to or smaller than 1.5 $\mu$m.

Filters (A) (prefilter), (B) (first main filter), and (C) (second main filter) are laminated in order of (A), (B), and (C) from blood inlet section 7I (7I') to blood outlet section 7O (7O') from the upper portion U side to the lower portion D side so as to constitute blood processing member 5. As shown in FIG. 9, blood processing member 5 can be disposed in housing 2 having blood inlet section 7I (7I') and blood outlet section 7O (7O').

For example, the number of sheets of (A) prefilter to be laminated is preferably 15 to 25, and the thickness thereof is preferably 2.1 to 4.2 mm; the number of sheets of (B) first main filter to be laminated is preferably 20 to 35, and the thickness thereof is preferably 2.0 to 5.1 mm; and the number of sheets of (C) second main filter to be laminated is preferably 5 to 15, and the thickness thereof is preferably 0.5 to 1.5 mm. By arranging the filters such that the average fiber diameter decreases in order of filter (A), filter (B), and filter (C) and that blood components having slightly different particle sizes are dealt with corresponding filters each having an appropriate fiber diameter in a case where blood containing those components is processed, overall filtering efficacy can be kept high.

(Material of housing 2 and outer frame sheet 3)

Housing 2 and outer frame sheet 3 are preferably constituted with sheets formed of a soft member made of a thermoplastic resin. Examples of preferable resins include soft polyvinyl chloride, polyurethane, a styrene-butadiene-styrene copolymer, a styrene-ethylene-butylene-styrene copolymer, a thermoplastic elastomer containing these as a main component, a hydrogenated material of a styrene-butadiene-styrene copolymer, a thermoplastic elastomer such as a styrene-isoprene-styrene copolymer or a hydrogenated material thereof, a mixture of a thermoplastic elastomer and a softener such as polyolefin or ethylene-ethyl acrylate, and an ethylene-vinyl acetate copolymer. These are members that can be fixed to each other by heat sealing.

[Materials of blood inlet section 7I (7I') and blood outlet section 7O (7O')]

Blood inlet section 7I (7I'), blood outlet section 7O (7O'), and the like are sites having an approximate pipe shape. As materials of blood inlet section 7I (7I') and blood outlet section 7O (7O'), materials that can be fixed to housing 2 and outer frame sheet 3 by heat sealing or the like are preferable. For example, in a case where the material of housing 2 and outer frame sheet 3 is soft polyvinyl chloride, the material of blood inlet section 7I (7I') and blood outlet section 7O (7O') is preferably semi-hard or hard polyvinyl chloride. Blood inlet section 7I (7I') and blood outlet section 7O (7O') are formed of a resin material harder than housing 2 or the like. Therefore, blood inlet section 7I (7I') and blood outlet section 7O (7O') can form a blood circuit by being easily connected to a tube constituting the blood circuit.

Hereinafter, specific embodiments of blood processing device 1 (Type I) will be described based on examples.

[Example A]

(Example 1)

As a blood processing device of Example 1, blood processing device 1 (Type I) assembled as below by the method described in FIGS. 3 to 8 was used.

Specifically, 40 sheets (n = 40) of nonwoven cloth (thickness d = 130 $\mu$m, fiber diameter 1 = 1.8 $\mu$m, length L of one side = 72 mm $\times$ 72 mm) made of polyethylene terephthalate were laminated and used as each of upper portion-side blood processing member 5/U and lower portion-side blood processing member 5/D (total thickness Td of each member = 5.2 mm).

Each of the members was fixed to an outer frame sheet by heat sealing by using a high-frequency welding machine.

Blood processing device 1 (Type I) was prepared as below. Each of a pair of blood processing members 5 on the upper portion U side and the lower portion D side was fixed to outer frame sheet 3 made of soft polyvinyl chloride, and outer frame sheet 3 was fixed to flexible housing 2 made of soft polyvinyl chloride. Blood inflow chamber 2IR was formed in the interior (into which whole blood will be supplied) of the pair of blood processing members 5 disposed to face each other, and blood outflow chamber 2OR (into which filtered blood will flow from blood processing member 5) was formed between the exterior (outside) of the pair of blood processing members (5/U and 5/D) and flexible housing 2.

As shown in FIG. 9, the interior of flexible housing 2 was divided into blood inflow chamber 2IR (into which whole blood will be supplied) and blood outflow chamber 2OR (into which filtered blood will flow from blood processing members 5) across blood processing members 5. Blood inlet section 7I was mounted on the proximal end side of blood inflow chamber 2IR, and blood outlet section 7O was mounted on the distal end side of blood outflow chamber 2OR. All of the fixing and the mounting described above was performed by heat sealing by using a high-frequency welding machine.

As blood for a filtration test, bovine blood was used. That is, a bovine blood-containing bag [bovine blood (400 ml) with an ACD solution (60 ml) as an anticoagulant] and an empty bag for accommodating filtered blood were prepared.

A tube was connected to each of blood inlet section 7I and blood outlet section 7O of the housing of blood processing device 1 constituted as above. The bovine blood-containing bag was connected to the upper end of the tube on the blood inlet section 7I side, the empty bag for accommodating filtered blood was connected to the lower end of the tube on the blood outlet section 7O side, and a head from the top of the bovine blood-containing bag to the top (blood inlet) of the empty bag was set to be 140 cm.

As a test, bovine blood was allowed to perform free fall from the bovine blood-containing bag and filtered through blood processing members 5. As a result, a time $\theta$ taken for the filtration of 400 mL of blood to be finished was 19 minutes.

(Comparative Example 1)

For a blood processing device (reference sign is not used intentionally) of Comparative Example 1, 60 sheets (n= 60) of nonwoven cloth (thickness d = 130 $\mu$m, fiber diameter 1 = 1.8 $\mu$m, length L of one side = 72 mm $\times$ 72 mm) made of polyethylene terephthalate was laminated and used as one blood processing member 5 (total thickness Td = 7.8 mm).

The blood processing device of Comparative Example 1 was prepared as below. Blood processing member 5 was fixed to outer frame sheet 3 made of soft polyvinyl chloride, and outer frame sheet 3 was fixed to flexible housing (2/U and 2/D) made of soft polyvinyl chloride. The interior of flexible housing (2/U and 2/D) was divided into blood inflow chamber 2IR and blood outflow chamber 2OR across blood processing member 5. Blood inlet section 7I was mounted on the proximal end side of blood inflow chamber 2IR, and blood outlet section 7O was mounted on the distal end side of blood outflow chamber 2OR. All of the fixing and the mounting described above were performed by heat sealing by using a high-frequency welding machine. The blood processing device of Comparative Example 1 has substantially the same form as blood processing device 201 which will be described later [housing (2/U and 2/D) was formed by three-dimensionally processing a thick sheet], except that the aforementioned flexible housing (2/U and 2/D) (sheet having a thickness of about 0.4 mm) was used as housing 2.

As blood for a filtration test, bovine blood prepared in the same manner as in Example 1 was used, and the same test as in Example 1 was performed. That is, as the test, the bovine blood was allowed to perform free fall from the bovine blood-containing bag as in Example 1 and filtered through the single blood processing member described above. As a result, a time $\theta$ taken for the filtration of 400 mL of blood to be finished was 37 minutes.

(Study of result)

The results of Example 1 and Comparative Example 1 were studied by comparison. In Comparative Example 1, the time $\theta$ taken for the filtration of 400 ml of blood to be finished was 37 minutes. However, in Example 1, the time $\theta$ taken for the filtration of blood to be finished was only 19 minutes. From this result, it was confirmed that Example 1, which is a specific embodiment of blood processing device 1 (Type I), can significantly reduce the filtration time required in a case where the blood processing device of the related art is used.

(Blood processing device 101 (Type III) of embodiment 3) (see FIGS. 13 to 22)

The key characteristics of blood processing device 101 of embodiment 3 of the present invention are as below. That is, in blood processing devices 1 and 1' of Types I and II described above, along the flow path connecting blood inlet sections 7I and 7I' and blood outlet sections 7O and 7O', blood processing member 5 is provided in each of the upper portion (U-side) and the lower portion (D-side) (a pair of upper and lower blood processing members 5/U and 5/D are disposed).

In contrast, in blood processing device 101 of Type III, each of the upper portion-side U area and the lower portion-side D area in the central flow path from blood inlet section 7I to blood outlet section 7O was further divided into a proximal end-side area (PE-side Area) and a distal end-side area (DE-side Area).

On the upper portion U side, a pair of (two) proximal end PE side-upper portion U side blood processing member 5/PEU and distal end DE side-upper portion U side blood processing member 5/DEU are disposed.

On the lower portion D side, a pair of (two) proximal end PE side-lower portion D side blood processing member 5/PED and distal end DE side-lower portion D side blood processing member 5/DED are disposed.

The pair of (two) blood processing members on the upper portion U side will be collectively described using reference signs (5/PEU and 5/DEU) or described as upper portion U-side first blood processing members (5/PEU and 5/DEU) in some cases.

Furthermore, the pair of (two) blood processing members on the lower portion D side will be collectively described using reference signs (5/PED and 5/DED) or described as lower portion D-side second blood processing members (5/PED and 5/DED) in some cases.

More specifically, a pair of upper and lower blood processing members 5 (on the upper portion U-side and the lower portion D-side) [referred to as first blood processing members (5/PEU and 5/PED)] are provided in the proximal end-side area (PE-Side Area), and the other pair of blood processing members 5 [referred to as second blood processing members (5/DEU and 5/DED)] are provided in the distal end-side area (DE-side Area) (see FIGS. 14 and 15) (hereinafter, "proximal end-side area" will be simply referred to as "proximal end side" in some cases, and "distal end-side area" will be simply referred to as "distal end side" in some cases).

The pair of upper and lower blood processing members (5/PEU and 5/PED) on the proximal end PE side will be referred to as a pair of upper and lower proximal end-side first blood processing members (5/PEU and 5/PED) in some cases, and the pair of upper and lower blood processing members (5/DEU and 5/DED) on the distal end DE side will be described as a pair of upper and lower distal end-side second blood processing members (5/DEU and 5/DED) in some cases.

Hereinafter, for the convenience of description, blood processing members will be described based on the pair of upper and lower proximal end-side first blood processing members (5/PEU and 5/PED) and the pair of upper and lower distal end-side second blood processing members (5/DEU and 5/DED).

On the proximal end side (PE-Side Area), the blood flowing into the device from blood inlet section 7I flows to the outside from the interior of blood processing members 5. On the distal end side (DE-side Area), the blood flows into the interior of blood processing member 5 from the outside and then flows to the outside from blood outlet section 7O.

Based on the above constitution, blood processing device 101 (Type III) will be more specifically described.

First, in blood processing device 101 of embodiment 3 of the present invention, just like blood processing member 5 shown in FIGS. 13 to 22, a pair of (two) members (5/PEU and 5/PED) are present on the upper portion U side and the lower portion D side of the proximal end PE side, and a pair of (two) members (5/DEU and 5/DED) are present on the upper portion U side and the lower portion D side of the distal end DE side. Accordingly, there are two pairs of (four) blood processing members in total. Hereinafter, how to use reference signs in this case will be defined, just in case.

In a case where "5/U" is used by adding a suffix "/U" after a reference sign "5", "5/U" means blood processing member 5/U on the upper portion U side.

Furthermore, in a case where "5/PEU" is used by adding a suffix "/PEU" after the reference sign "5", "5/PEU" means blood processing member 5/PEU on the upper portion U side of the proximal end PE side (hereinafter, described as "proximal end PE side-upper portion U side" in some cases).

Likewise, "5/DEU" means blood processing member 5/DEU on the upper portion U side of the distal end DE side.

In order to simplify the name and the reference sign of each member, in some cases, a pair of (two) blood processing member 5/PEU on the proximal end PE side-upper portion U side and blood processing member 5/PED on the proximal end PE side-lower portion D side will be collectively described using the reference signs (5/PEU and 5/PED) or collectively described as blood processing member 5/PE on the proximal end PE side by using a reference sign.

In some cases, blood processing member 5/PE on the proximal end PE side will be described as proximal end-side first blood processing member, and blood processing member 5/DE on the distal end DE side will be described as distal end-side second blood processing member.

In addition, two pairs of (four) blood processing member 5/PEU on the upper portion U side of the proximal end PE side, blood processing member 5/PED on the lower portion D side of the proximal end PE side, blood processing member 5/DEU on the upper portion U side of the distal end DE side, and blood processing member 5/DED on the lower portion D side of the distal end DE side will be collectively described using reference signs (5/PEU, 5/PED, 5/DEU, 5/DED) in some cases. Furthermore, these will be collectively described using reference signs (5/PEU, PED, DEU, and DED) in some cases.

Furthermore, for the convenience of description, sometimes all of the plurality of members will be simply described as "blood processing member 5" by using only the initial reference sign (without the last reference sign, /, PE, U, and the like).

For two pairs of (four) blood processing members (5/PEU, 5/PED, 5/DEU, and 5/DED), four pairs of (eight) upper and lower outer frame sheets 3 are present. Therefore, outer frame sheets 3 are described as below.

For example, outer frame sheet 3 on the upper portion U side of blood processing member 5/PEU of "proximal end PE side-upper portion U side" is described using a reference sign "3/PEUU", and outer frame sheet 3 on the lower portion D side is described using a reference sign "3/PEUD". Hereinafter, outer frame sheet 3 will be described as shown in FIG. 15 in the same manner.

Proximal end side (PE-Side Area)

A pair of upper outer frame sheet 3/PEUU and lower outer frame sheet 3/PEUD on the proximal end side-upper portion side will be described as first of the first outer frame sheets (3/PEUU and 3/PEUD) in some cases.

A pair of upper outer frame sheet 3/PEDU and lower outer frame sheet 3/PEDD on the proximal end side-lower portion side will be described as second of the first outer frame sheets (3/PEDU and 3/PEDD) in some cases.

Distal end side (DE-side Area)

A pair of upper outer frame sheet 3/DEUU and lower outer frame sheet 3/DEUD on the distal end side-upper portion side will be described as first of the second outer frame sheets (3/DEUU and DEUD) in some cases.

A pair of upper outer frame sheet 3/DEDU and lower outer frame sheet 3/DEDD on the distal end side-lower portion side will be described as second of the second outer frame sheets (3/DEDU and 3/DEDD) in some cases.

The names and the reference signs of other members will be simplified by the same description method as that used for blood processing member 5.

Other members (except for housing 2 and sheet SH) will be described using reference signs in the same manner as that used for blood processing member 5, and the names and the reference signs of those other members will be simplified by the same description method as that used for blood processing members 5.

In some cases, housing 2 (sheet SH) on the upper portion U side will be described as first housing (sheet), and housing 2 (sheet SH) on the lower portion D side will be described as second housing (sheet).

Regarding how to use reference signs in drawings, for example, in a case where there are two pairs of (four) members just like blood processing member 5 [that is, a pair of upper and lower members (5/PEU and 5/PED) in the proximal end-side area (PE-Side Area) and a pair of upper and lower members (5/DEU and 5/DED) in the distal end-side area (DE-Side Area)], and a plurality of those (members) are shown in a drawing, a plurality of reference signs such as "5/PEU and 5/DEU" are used. Reference signs are not used for those not shown in the drawing.

As shown in FIGS. 13 to 22, blood processing device 101 (Type III) of embodiment 3 has flexible housing 2 formed of flexible sheet SH (hereinafter, simply described as housing 2) and blood processing member 5 installed in the interior of housing 2.

Housing 2 includes first housing 2/U on the upper portion U side and second housing 2/D on the lower portion D side.

On the proximal end PE side of housing 2, tubular blood inlet section 7I is mounted on one end portion of the approximate center in the longitudinal direction.

Furthermore, on the distal end DE side of housing 2, tubular blood outlet section 7O is mounted on one end portion of the approximate center in the longitudinal direction.

Similarly to blood processing devices 1 and 1', it is preferable that tubular blood inlet section 7I and blood outlet section 7O are formed of a resin which is harder than flexible housing 2 formed of flexible sheet SH. In addition, similarly to blood processing devices 1 and 1', in blood inlet section 7I and blood outlet section 7O, inlet side hole 8I and outlet side hole 8O are formed.

Similarly to blood processing devices 1 and 1', blood processing member 5 is constituted with a pair of upper and lower proximal end-side first blood processing members (5/PEU and 5/PED), which are in the proximal end PE-side area and formed by laminating a plurality of sheets of nonwoven cloth having a predetermined thickness, and a pair of upper and lower distal end-side second blood processing members (5/DEU and 5/DED), which are in the distal end DE-side and formed by laminating a plurality of sheets of nonwoven cloth having a predetermined thickness.

As illustrated in FIGS. 13 to 22, blood processing device 101 of embodiment 3 have the following characteristics (compared to blood processing devices 1 and 1'). Note that the size of the members (particularly, the thickness of the blood processing member) and the like illustrated in FIGS. 13 to 22 are slightly exaggerated to facilitate understanding of the characteristics of blood processing device 101.

(a) First, in the interior of flexible housing 2 (2/U and 2/D), a pair of (two) proximal end PE-side first blood processing members (5/PEU and 5/PED) are disposed in the proximal end PE-side area, and a pair of (two) distal end DE-side second blood processing members (5/DEU and 5/DED) are disposed in the distal end DE-side area. In this way, two pairs (four) blood processing members (5/PEU, PED, DEU, and DED) are disposed in blood processing device 101, and this is one of the characteristic of blood processing device 101. That is, in blood processing device 1 (Type I), along the line connecting blood inlet section 7I and blood outlet section 7O, a pair of (two) blood processing members (so-called filtration units) are disposed on the upper side and the lower side thereof. In contrast, in blood processing device 101 (Type III), the blood processing members are subdivided into a total of two pairs of (four) blood processing members such that a pair of upper and lower blood processing members are disposed in the proximal end-side area (PE-side Area) and a pair of upper and lower blood processing members are disposed in the distal end-side area (DE-side Area).

(b) In blood processing device 101, in a case where each of blood processing members 5 is seen along the direction of upper portion U and the direction of lower portion D, each of the proximal end-side area (PE-side Area) and the distal end-side area (DE-side Area) is in the form of an approximate triangle (see FIG. 13, in the drawing, 5/PEU is a blood processing member on the upper portion U side of the proximal end PE side, and 5/DEU is a blood processing member on the upper portion U side of the distal end D side).

(c) Furthermore, for example, in blood processing device 101, between (on the inside of) the pair of (two) upper and lower first blood processing members (5/PEU and 5/PED) in the proximal end-side area (PE-side Area), proximal end-side first blood inflow chamber 2IR/PE is formed; and between (on the inside of) the pair of (two) upper and lower second blood processing members (5/DEU and 5/DED) in the distal end-side area (DE-side Area), distal end-side second blood inflow chamber 2IR/DE is formed. In this way, blood inflow chamber 2IR/PE on the proximal end PE side and blood inflow chamber 2IR/DE on the distal end DE side are formed. Here, first blood inflow chamber 2IR/PE on the proximal end PE side and second blood inflow chamber 2IR/DE on the distal end DE side are separated from each other by outer frame sealing portion 3S and do not directly communicate with each other.

(d) In blood processing device 101, between the inside of the proximal end PE-side area (PE-side Area) of housing 2 and the outside of first blood processing members (5/PEU and 5/PED) in the same area, proximal end-side first blood outflow chambers (2OR/PEU, 2OR/PED, and 2OR/PES) are formed (see FIGS. 13 and 14); and between the inside of the distal end DE-side area (DE-side Area) of housing 2 and the outside of second blood processing members (5/DEU and 5/DED) in the same area, distal end-side second blood outflow chambers (2OR/DEU, 2OR/DED, and 2OR/DES) are formed (see FIGS. 13 and 14).

(e) In the actual operation of supplying blood to blood processing device 101 constituted as above and filtering the blood, first, as a first step, blood is introduced into proximal end-side first blood inflow chamber 2IR/PE from blood inlet section 7I on the proximal end PE side, and filtered through the pair of (two) blood processing members (5/PEU and 5/PED) in the proximal end PE-side area (PE-side Area) toward the outside of the blood processing members from the inside thereof (see FIG. 14).

(f) Then, as a second step, the blood passes through proximal end-side first blood outflow chambers (2OR/PEU, 2OR/PED, and 2OR/PES) in the proximal end PE-side area (PE-side Area) and distal end-side second blood outflow chambers (2OR/DEU, 2OR/DED, and 2OR/DES) in the distal end DE side (DE-side Area), is filtered through second blood processing members (5/DEU and 5/DED) on the distal end DE side toward the outside of the blood processing members from the inside thereof, passes through the distal end-side second blood inflow chamber 2IR/DE in the distal end DE-side area (DE-side Area), and is discharged through blood outlet section 7O (see FIG. 14).

(Assembly process (assembly) of blood processing device 101)

Hereinafter, an aspect of an Example of blood processing device 101 will be described based on an example of an assembly process.

A plurality of sheets (specifically, approximately more than ten sheets to dozens of sheets) of nonwoven cloth having a predetermined thickness are laminated, thereby preparing two pairs of (four) blood processing members 5 (5/PEU, 5/PED, 5/DEU, and 5/DED) [four blood processing members in total constituted with one pair in the proximal end PE-side area (PE-side Area) and one pair in the distal end DE-side area (DE-side Area)].

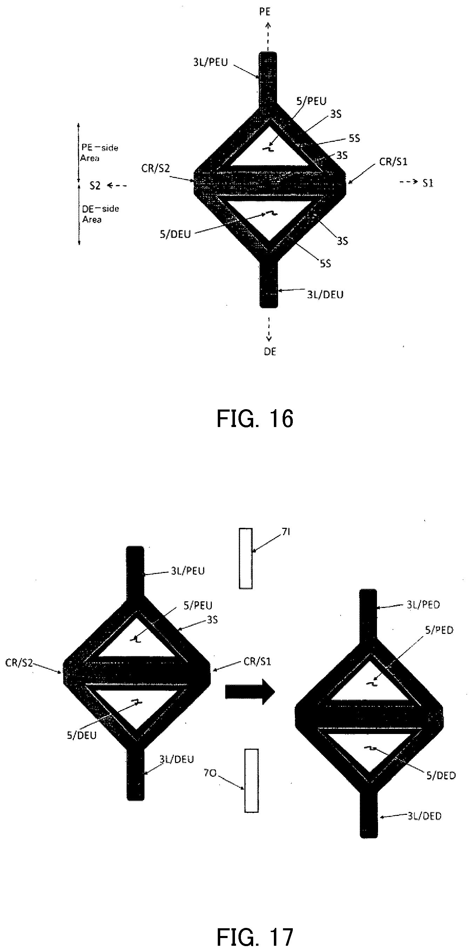

As illustrated in FIG. 15 (FIG. 16), blood processing members 5 are fixed to four pairs of (eight) outer frame sheets (3/PEUU, 3/PEUD, 3/PEDU, and 3/PEDD; 3/DEUU, 3/DEUD, 3/DEDU, and 3/DEDD).

That is, in the proximal end PE-side area (PE-side Area), as shown in FIG. 15, first blood processing member (5/PEU) on the upper portion side is fixed between the inner edges of a pair of (two) upper and lower first of the first outer frame sheets (3/PEUU and 3/PEUD) (inner edges refer to surfaces facing each other across blood processing member 5; the same is true for the following description), and first blood processing member (5/PED) on the lower portion side is fixed between the inner edges of a pair of (two) upper and lower second of the first outer frame sheets (3/PEDU and 3/PEDD).

Likewise, in the distal end DE-side area (DE-side Area), as shown in FIG. 15, second blood processing member (5/DEU) on the upper portion side is fixed between the inner edges of a pair of (two) upper and lower first of the second outer frame sheets (3/DEUU and 3/DEUD), and second blood processing member (5/DED) on the lower portion side is fixed between the inner edges of a pair of (two) upper and lower second of the second outer frame sheets (3/DEDU and 3/DEDD).

Similarly to the cases of Types I and II, fixing of the above members can be performed by welding by means of external heating such as heat sealing, internal heating by a high frequency or an ultrasonic wave, and the like.

Hereinafter, the fixing portions of blood processing member 5 and outer frame sheet 3 will be referred to as "sealing portion 5S" in some cases. Non-fixing portions on the inside of blood processing member 5 and outer frame sheet 3 are elements substantially functioning as blood filters, and will be referred to as "filtration section 5F" in some cases.

Among filtration sections 5F, the surface on the blood inflow chamber 2IR side will be referred to as "inner side filtration section 5FI" in some cases, and the surface on the blood outflow chamber 2OR side will be referred to as "outer side filtration section 5FO" in some cases. Similarly to blood processing member 5, inner side filtration section 5FI and outer side filtration section 5FO make a pair (at two sites).

The outer edges of four pairs of (eight) outer frame sheets 3 (3/PEUU, 3/PEUD, 3/PEDU, 3/PEDD, 3/DEUU, 3/DEUD, 3/DEDU, and 3/DEDD) are fixed as well. Hereinafter, these fixing portions will be referred to as "outer frame sealing portion 3S" in some cases.

The distal end DE side of the pair of (two) upper and lower proximal end-side first blood processing members (5/PEU and 5/PED) and the proximal end PE side of the pair of (two) upper and lower distal end-side second blood processing members (5/DEU and 5/DED) are connected to each other in a longitudinal L direction by outer frame sealing portion 3S (see FIGS. 16 and 17).

Furthermore, a pair of (two) upper and lower proximal end-side first lengthened sections (3L/PEU and 3L/PED) and a pair of (two) upper and lower distal end-side second lengthened sections (3L/DEU and 3L/DED) are fixed to proximal end-side first of the first outer frame sheet 3/PEUU and proximal end-side second of the first outer frame sheet 3/PEDD as well as distal end-side first of the second outer frame sheet 3/DEUU and distal end-side second of the second outer frame sheet 3/DEDD. Note that lengthened sections of these outer frame sheets may be fixed from the first by means of integral molding, or may be fixed by means of welding by internal heating described above.

That is, in the proximal end PE-side area (PE-side Area), lengthened section 3L/PEU on the proximal end-upper portion side is fixed to the proximal end PE side of upper (first of the first) outer frame sheet 3/PEUU on the proximal end-upper portion side, and lengthened section 3L/PED on the proximal end-lower portion side is fixed to the proximal end PE side of lower (second of the first) outer frame sheet 3/PEDD on the proximal end side-lower portion side.

In addition, in the distal end DE-side area (DE-side Area), lengthened section 3L/DEU on the distal end-upper portion side is fixed to the distal end DE side of upper (first of the second) outer frame sheet 3/DEUU on the distal end-upper portion side, and lengthened section 3L/DED on the distal end-lower portion side is fixed to the distal end DE side of lower (second of the second) outer frame sheet 3/DEDD on the distal end side-lower portion side.

Blood inlet section 7I is fixed between the pair of (two) first lengthened sections (3L/PEU and 3L/PED), and blood outlet section 7O is fixed between the pair of (two) second lengthened sections (3L/DEU and 3L/DED).

As illustrated in FIG. 14, blood processing member 5 is further compressed in the fixing portions (sealing portions 5S) of blood processing member 5 and outer frame sheet 3 than in the non-fixing portions (filtration sections 5F) on the inside of sealing portions 5S.

Then, as illustrated in FIG. 17 (see FIG. 14 as well), the inner side filtration sections of the pair of upper and lower blood processing members (5/PEU and 5/PED) in the proximal end-side area (PE-side Area) caused to face the inner side filtration sections of the pair of upper and lower blood processing members (5/DEU and 5/DED) in the distal end-side area (DE-side Area). That is, the blood processing members are laminated in a state where U-side inner side filtration section (5FI/PEU) in the proximal end PE-side area (PE-side Area) faces D-side inner side filtration section (5FI/PED) in the proximal end PE-side area (PE-side Area) and U-side inner side filtration section (5FI/DEU) in the distal end DE-side area (DE-side Area) faces D-side inner side filtration section (5FI/DED) in the distal end DE-side area (DE-side Area).

In the proximal end PE-side area (PE-side Area), upper portion-side lengthened section 3L/PEU, blood inlet section 7I, and lower portion-side lengthened section 3L/PED are disposed.

In the distal end DE-side area (DE-side Area), upper portion-side lengthened section 3L/DEU, blood outlet section 7O, and lower portion-side lengthened section 3L/DED are disposed.

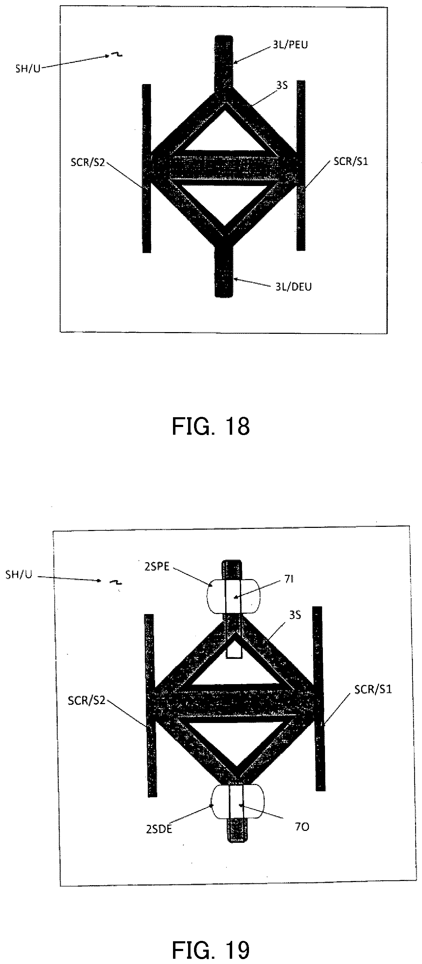

Thereafter, as illustrated in FIG. 18, the pair of upper and lower proximal end-side first blood processing members (5/PEU and 5/PED) in the proximal end PE-side area (PE-side Area) and the pair of upper and lower distal end-side second blood processing members (5/DEU and 5/DED) in the distal end DE-side area (DE-side Area) are covered with a pair of upper and lower sheets SH/U and SH/D (upper and lower sheets SH/U and SH/D become housing 2/U on the upper portion U side and housing 2/D on the lower portion D side). Subsequently, corners CR/S1 and CR/S2 on the first side portion S1 side and the second side portion S2 side are fixed by heat sealing or the like. Hereinafter, the fixing portions will be referred to as "corner sealing portions (SCR/S1 and S2)" in some cases.

Then, as illustrated in FIG. 19, the pair of upper and lower first lengthened sections (3L/PEU and 3L/PED) and blood inlet section 71 in the proximal end PE-side area (PE-side Area), the pair of upper and lower second lengthened sections (3L/DEU and 3L/DED) and blood outlet section 7O in the distal end DE-side area (DE-side Area), and the peripheries (outer edges and vicinities) of these are fixed to the pair of upper and lower sheets SH/U and SH/D by heat sealing or the like.

Hereinafter, the fixing portions will be referred to as "proximal end-side sealing portion 2SPE" and "distal end-side sealing portion 2SDE" in some cases.

For example, the pair of upper and lower first lengthened sections (3L/PEU and 3L/PED) and blood inlet section 71 in the proximal end PE-side area (PE-side Area) may be held by two sheets SH/U and SH/D and fixed, and then the pair of upper and lower second lengthened sections (3L/DEU and 3L/DED) and blood outlet section 7O in the distal end DE-side area (DE-side Area) may be held by two sheets SH/U and SH/D and fixed. Alternatively, the pair of upper and lower first lengthened sections (3L/PEU and 3L/PED) and blood inlet section 71 in the proximal end PE-side area (PE-side Area) as well as the pair of upper and lower second lengthened sections (3L/DEU and 3L/DED) and blood outlet section 7O in the distal end DE-side area (DE-side Area) may be simultaneously fixed to two sheets SH/U and SH/D.

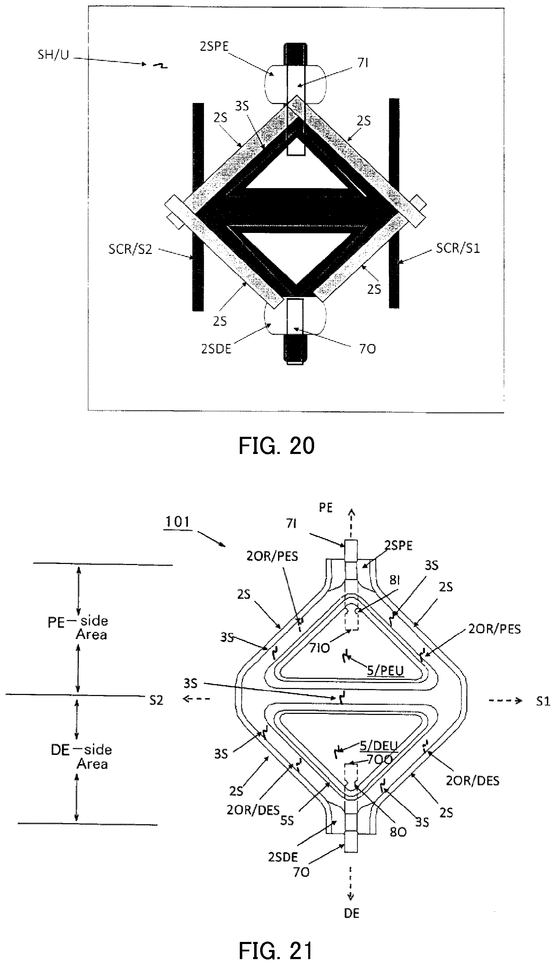

Thereafter, as illustrated in FIG. 20, outer frame sealing portions 3S on the outer edges of 4 sides (oblique sides) of blood processing member 5 are fixed to sheet SH/U and sheet SH/D by heat sealing. Hereinafter, the fixing portions will be referred to as "sealing portion 2S" in some cases.

Last, unnecessary sheets SH/U and SH/D on the outer edges are removed (cut) from each of sealing portions 2S and SCR, thereby completing blood processing device 101 described in FIG. 21.

In order to easily remove (cut) SH/U and SH/D from each of sealing portions 2S and SCR, the tip of a heat sealing electrode or the like may be made approximately triangular such that the tip welding portion becomes thick after heat sealing. Alternatively, by causing a cutter to be adjacent to the heat sealing electrode or the like, a thick portion (cut line) can be formed in each of sealing portions 2S and SCR.

(Form of blood processing device 101 (Type III))

The form of blood processing device 101 will be described. As will be specifically described below, the sites on which blood inlet section 7I and blood outlet section 7O are mounted are fixed by lengthened sections 3L of outer frame sheet 3. That is, for example, as shown in FIG. 14, in the proximal end PE-side area (PE-side Area), at the site on which blood inlet section 71 is mounted, from the upper portion U side (U-side) to the lower portion D side (D-side), upper portion-side (U-side) housing 2/U, first lengthened section 3L/PEU on the upper portion side (U-side), blood inlet 71, first lengthened section 3L/PED on the lower portion side (D-side), and housing 2/D on the lower portion side (D-side) are fixed in this order.

In the proximal end PE-side area (PE-side Area), first blood processing member 5/PEU on the upper portion side (U-side) is fixed to first lengthened section 3L/PEU on the upper portion side through the outer frame sealing portion 3S of the pair of upper and lower first of the first outer frame sheets (3/PEUU and 3/PEUD).

In the proximal end PE-side area (PE-side Area), first blood processing member 5/PED on the lower portion side (D-side) is fixed to first lengthened section 3L/PED on the lower portion side (D-side) through the outer frame sealing portion 3S of the pair of upper and lower second of the first outer frame sheets (3/PEDU and 3/PEDD).

In contrast, in the distal end DE-side area (DE-side Area), as shown in FIG. 14, at the site on which blood outlet section 7O is mounted, from the upper portion U side (U-side) to the lower portion D side (D-side), upper portion-side housing 2/U, second lengthened section 3L/DEU on the upper portion side, blood inlet section 71, second lengthened section 3L/DED on the lower portion side, and lower portion-side housing 2/D are fixed in this order.

In the distal end DE-side area (DE-side Area), second blood processing member 5/DEU on the upper portion side (U-side) is fixed to second lengthened section 3L/DEU on the upper portion side through outer frame sealing portion 3S of the pair of upper and lower first of the second outer frame sheets (3/DEUU and 3/DEUD).

In the distal end DE-side area (DE-side Area), second blood processing member 5/DED on the lower portion side (D-side) is fixed to second lengthened section 3L/DED on the lower portion side through outer frame sealing portion 3S of the pair of upper and lower second of the second outer frame sheets (3/DEDU and 3/DEDD).

The distal end DE side of first blood processing members (5/PEU and 5/PED) in the proximal end PE-side area and the proximal end PE side of second blood processing members (5/DEU and 5/DED) in the distal end DE-side area are fixed in the following manner as shown in FIG. 15.

That is, the distal end DE side and the proximal end PE side described above are fixed through outer frame sealing portion 3S on the distal end DE side of lower first of the first outer frame sheet 3/PEUD and upper second of the first outer frame sheet 3/PEDU and outer frame sealing portion 3S on the proximal end PE side of lower first of the second outer frame sheet 3/DEUD and upper second of the second outer frame sheet 3/DEDU (see FIGS. 16 and 21 as well).

As shown in FIG. 14, the distal end DE side of blood inlet section 71 in the proximal end PE-side area (PE-side Area) is stuck into the interior of proximal end-side first blood inflow chamber 2IR/PE.

In blood inlet section 71, inlet side hole 8I is formed at a position closer to the proximal end PE side than inlet opening 71O on the extreme distal end DE side. This point is the same as Types I and II.

Inlet side hole 8I is formed in proximal end-side first blood inflow chamber 2IR/PE, at a position which is in the vicinity of the proximal end PE side of inner side filtration sections (5FI/PEU and 5FI/PED) of proximal end-side first blood processing members (5/PEU and 5/PED). Inlet side hole 8I may be formed at 2 sites or formed at 3 to 4 sites. This point is also the same as Types I and II.

(Function of side hole 8)

As described above regarding Types I and II, in a case where blood flows into blood processing device only from inlet opening 71O of blood inlet section 71, it is apprehended that due to the compression of blood processing member 5/PEU on the upper portion side and blood processing member 5/PED on the lower portion side, distal end DE side of blood inlet section 71 may be crushed, and a blood flow path could not be secured. However, in a case where inlet side hole 8I is provided, even though the distal end DE side of blood inlet section 7I is crushed and blocked, blood can stably flow into the blood processing device from inlet side hole 8I.

Meanwhile, the proximal end PE side of blood outlet section 7O on the distal end DE side is stuck into the interior of distal end-side second blood inflow chamber 2IR/DE. In blood outlet section 7O, outlet side hole 8O is formed at a position closer to the distal end DE side than opening 7OO on the extreme proximal end PE side.

Outlet side hole 8O is formed in distal end-side second blood inflow chamber 2IR/DE, at a position which is in the vicinity of the distal end DE side of inner side filtration sections (5FI/DEU and 5FI/DED) of the distal end-side second blood processing members (5/DEU and 5/DED). Outlet side hole 8O may be formed at 2 sites or formed at 3 to 4 sites. This point is the same as Types I and II.

In a case where blood flows out of the blood processing device only from outlet opening 7OO of blood outlet section 7O, it is apprehended that due to the compression of blood processing member 5/DEU on the upper portion side and blood processing member 5/DED on the lower portion side, proximal end PE side of blood outlet section 7O may be crushed, and a blood flow path could not be secured. However, in a case where outlet side hole 8O is provided, even though the proximal end PE side of blood outlet section 7O is crushed, blood can stably flow out of the blood processing device from outlet side hole 8O.

(Internal structure of housing 2 of blood processing device 101 (Type III))

The internal structure of housing 2 will be specifically described.

The outer edges of the pair of first blood processing members (5/PEU and 5/PED) in the proximal end PE-side area (PE-side Area) are fixed to the inner edges of the pair of first outer frame sheets (3/PED and 3/PEU), and the outer edges of the pair of first outer frame sheets (3/PEU and 3/PED) are fixed to housing 2.

In contrast, the outer edges of the pair of second blood processing members (5/DEU and 5/DED) in the distal end DE-side area (DE-side Area) are fixed to the inner edges of the pair of second outer frame sheets (3/DEU and 3/DED), and the outer edges of the pair of second outer frame sheets (3/DEU and 3/DED) are fixed to housing 2.

In the proximal end PE-side area (PE-side Area), between the lower portion D side of blood processing member 5/PEU on the upper portion side (U-side) and the upper portion U side of the blood processing member 5/PED on the lower portion side (D-side), the approximate center of housing 2 in the longitudinal direction, that is, a line (area) ("flow path-forming area") connecting the distal end DE side of blood inlet section 71 and the proximal end PE side of blood outlet section 7O in series forms a space. This space becomes blood inflow chamber 2IR/PE in THE proximal end PE-side area (PE-side Area).

In the distal end DE-side area (DE-side Area), between the lower portion D side of blood processing member 5/DEU on the upper portion side (U-side) and the upper portion U side of the blood processing member 5/DED on the lower portion side (D-side), the approximate center of housing 2 in the longitudinal direction, that is, a line (area) ("flow path-forming area") connecting the distal end DE side of blood inlet section 7I and the proximal end PE side of blood outlet section 7O in series forms a space. This space becomes blood inflow chamber 2IR/DE in the distal end DE-side area.

Between the inner periphery of the upper portions-side housing 2/U as well as the lower portion D-side housing 2/D and the outer periphery of blood processing members (5/PEU and 5/DEU) on the upper portion side as well as blood processing members (5/PED and 5/DED) on the lower portion side (outer side filtration sections 5FO/PEU, 5FO/PED, 5FO/DEU, and 5FO/DED), spaces are formed. These spaces become blood outflow cambers (2OR/PEU, 2OR/PED, 2OR/PES, 2OR/DEU, 2OR/DED, and 2OR/DES) (see FIG. 14).

Blood inlet section 71 communicates with first blood inflow chamber 2IR/PE in the proximal end PE-side area (PE-side Area), and blood outlet section 7O communicates with second blood inflow chamber 2IR/DE in the distal end DE-side area (see FIG. 14).

(Flow of blood in blood processing device 101 (Type III))

Next, the flow of blood in blood processing device 101 in an actual usage state will be described.

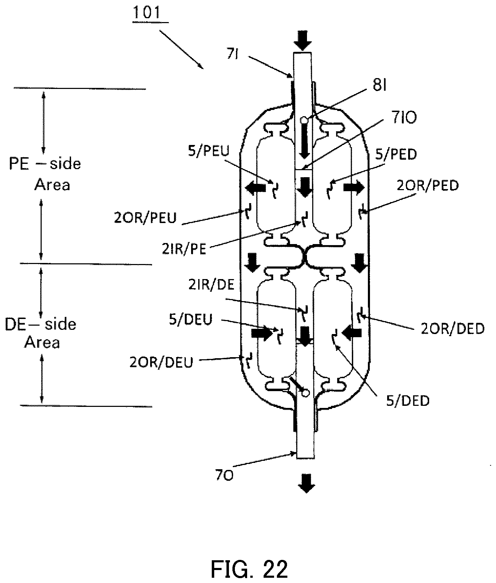

As illustrated in FIG. 22 (see the reference signs in FIG. 14 as well), basically, similarly to blood processing devices 1 and 1', blood processing device 101 is used in a state where the proximal end PE side thereof is the top and the distal end DE side thereof is the bottom such that blood flows by free fall (gravitational head).

Blood from the proximal end PE side of blood inlet section 7I passes through inlet opening section 7IO of the distal end DE side and inlet side hole 8I and flows into blood inflow chamber 2IR/PE in the proximal end PE-side area. Due to the inflow of the blood in the proximal end PE-side area, the internal pressure of blood inflow chamber 2IR/PE becomes positive.

The blood in blood inflow chamber 2IR/PE in the proximal end PE-side area is further pushed due to the positive pressure and gravity, passes through inner side filtration sections 5FI/PEU and 5FI/PED of blood processing members 5/PEU and 5/PED in the proximal end PE-side area and then through the outer side filtration sections 5FO/PEU and 5FO/PED, and moves into the blood outflow chambers 2OR/PEU, 2OR/PED, and 2OR/PES in the proximal end PE-side area (for 2OR/PES, see FIGS. 13 and 21).

Due to the positive pressure and gravity, the blood in blood outflow chambers 2OR/PEU, 2OR/PED, and 2OR/PES moves to blood outflow chambers 2OR/PED and 2OR/PES in the distal end DE-side area.

Due to the positive pressure and gravity, the blood in blood outflow chambers 2OR/PED and 2OR/PES passes through outer side filtration sections 5FO/DEU and 5FO/DED of blood processing members 5/DEU and 5/DED in the distal end DE-side area and then through inner side filtration sections 5FI/DEU and 5FI/DED, and flows into blood inflow chamber 2IR/DE in the distal end DE-side area.

The blood in blood inflow chamber 2IR/DE in the distal end DE-side area passes through outlet opening 7OO and outlet side hole 8O and is discharged to the outside from blood outlet section 7O.

In this way, blood processing members (5/PEU, PED, DEU, and DED) of Type III secure an effective filtration area much larger than that of Types I and II. That is, in Type III, two pairs of (four) blood processing members (5/PEU, PED, DEU, and DED) are disposed which are further subdivided compared to the pair of blood processing members (5/U and D) in Types I and II. Therefore, Type III is expected to more significantly reduce the filtration time compared to blood processing devices 1 and 1' in PTLS 1 to 4 and embodiments 1 and 2, and as a result, a leukocyte removal efficiency is expected to be improved.

Blood processing member 5 [proximal end-side first blood processing members (5/PEU and 5/PED) and distal end-side second blood processing members (5/DEU and 5/DED)] of blood processing device 101 (Type III) of embodiment 3 is the same as blood processing member 5 of blood processing devices 1 and 1' of embodiment 1 (Type I) and embodiment 2 (Type II), in terms of the constituent material, the material, the fiber diameter, the number of sheets to be laminated, the thickness, the form of lamination, the lamination direction, the lamination order, the material of housing 2 and outer frame sheet 3, and the material of blood inlet section 7I and blood outlet section 7O.

Preferable examples of embodiment 3 are as below.

(Preferable embodiment of Type III)

Basically, it is preferable that blood processing member 5 of Type III is formed by laminating a plurality of sheets of the same nonwoven cloth as that in Types I and II such that blood processing member 5 of Type III has a predetermined thickness. In this case, it is particularly preferable that the number of sheets of nonwoven cloth to be laminated to form a pair of (two) proximal end-side first blood processing members (5/PEU and 5/PED) is basically about 3 to 20 and preferably about 5 to 10. Furthermore, it is particularly preferable that the number of sheets of nonwoven cloth to be laminated to form a pair of (two) distal end-side second blood processing members (5/DEU and 5/DED) is about 10 to 40 and preferably about 20 to 30. An average fiber diameter D of proximal end-side first blood processing members (5/PEU and 5/PED) is a relatively large diameter which is about equal to or greater than 5.0 $\mu$m and equal to or smaller than 10.0 $\mu$m, for example. An average fiber diameter D of distal end-side second blood processing members (5/DEU and 5/DED) is a relatively small diameter which is about equal to or greater than 1.0 $\mu$m and equal to or smaller than 5.0 $\mu$m, for example.

In a case where the above constitution is adopted, proximal end-side first blood processing members (5/PEU and 5/PED) can function as so-called prefilters removing relatively big blood components, and distal end-side second blood processing members (5/DEU and 5/DED) can function as main filters.

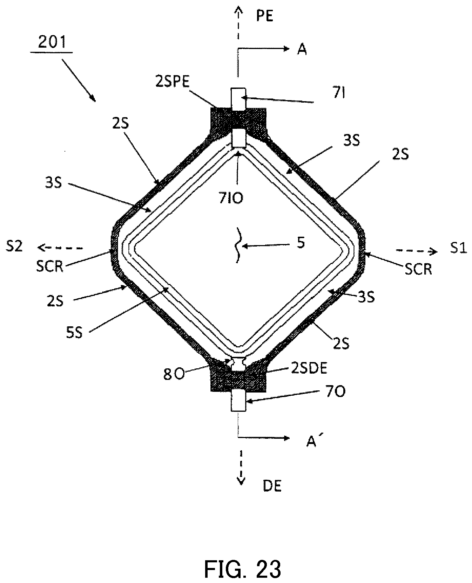

(Blood processing device 201 (Type IV) of embodiment 4) (see FIGS. 23 to 31)

As shown in FIGS. 23 to 31, blood processing device 201 (Type IV) of the present invention has flexible housing 2 (hereinafter, simply described as housing 2) obtained by processing a thick flexible sheet into a three-dimensional shape and blood processing member 5 installed in the interior of housing 2.

Housing 2 is constituted with housing 2/U on the upper portion U side that is processed to have a three-dimensional shape and housing 2/D on the lower portion D side that is processed to have a three-dimensional shape.

It is preferable that upper portion-side housing 2/U and lower portion-side housing 2/D are prepared by three-dimensionally processing a flexible polyvinyl chloride sheet thicker than a flexible sheet (thickness of about 0.4 mm) used, for example, in a general flexible housing (see comparative examples which will be described later).

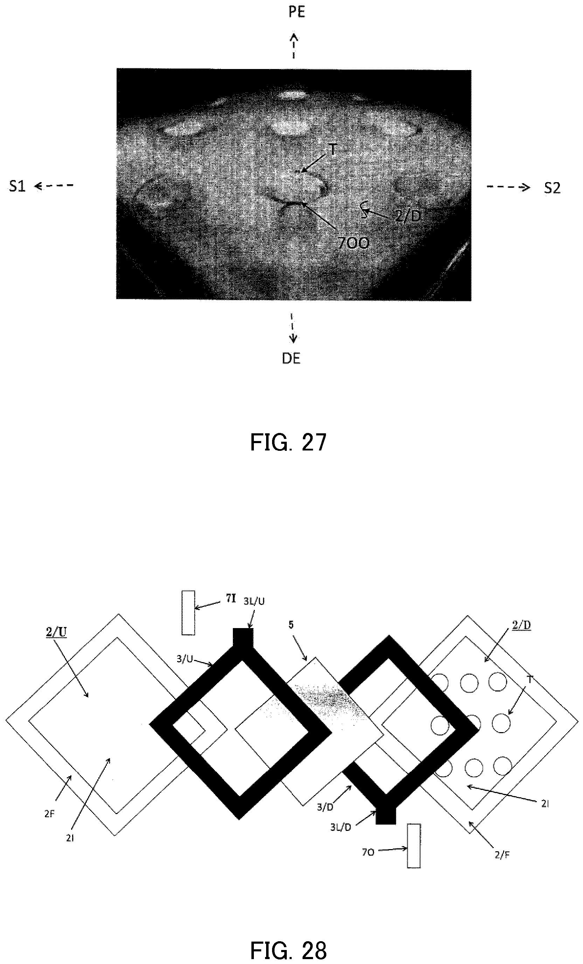

As shown in FIG. 28, a characteristic of blood processing device 201 is that upper portion-side housing 2/U and lower portion-side housing 2/D are formed such that interior area 2I thereof rises from outer edge portion 2F, and a plurality of projections T are provided in interior 2I of lower portion-side housing 2/D.

"Three-dimensionally processing (processing into a three-dimensional shape)" means, for example, that the aforementioned thick sheet is processed to have the shape of upper portion-side housing 2/U and lower portion-side housing 2/D, by using a mold or the like used for molding the shape (grooves, projections T, or the like) of upper portion-side housing 2/U and lower portion-side housing 2/D.

As the flexible sheet used in upper portion-side housing 2/U and lower portion-side housing 2/D, for example, a flexible sheet having a thickness of about equal to or greater than 0.8 mm and equal to or smaller than 1.5 mm can be suitably used.

A characteristic of lower portion-side housing 2/D is that a thick sheet is used as 2/D. However, the thicker the 2/D is, the more difficult it is for 2/D to be deformed in a case where the internal pressure of blood outflow chamber 2OR becomes negative. Therefore, thicker 2/D is preferable. That is, it is preferable that lower portion-side housing 2/D is thick, because then the inner wall of 2/D is hardly deformed and moves toward outer side filtration section 5FO.

Here, in a case where lower portion-side housing 2/D is too thick and has a thickness exceeding 1.5 mm for example, it is difficult to perform the aforementioned three-dimensional processing. Therefore, extremely thick 2/D is not preferable.

In contrast, in a case where 2/D is too thin and has a thickness less than 0.8 mm for example, because 2/D is formed of a flexible material, the deformation caused by the negative pressure cannot be inhibited. Therefore, extremely thin 2/D is not preferable.

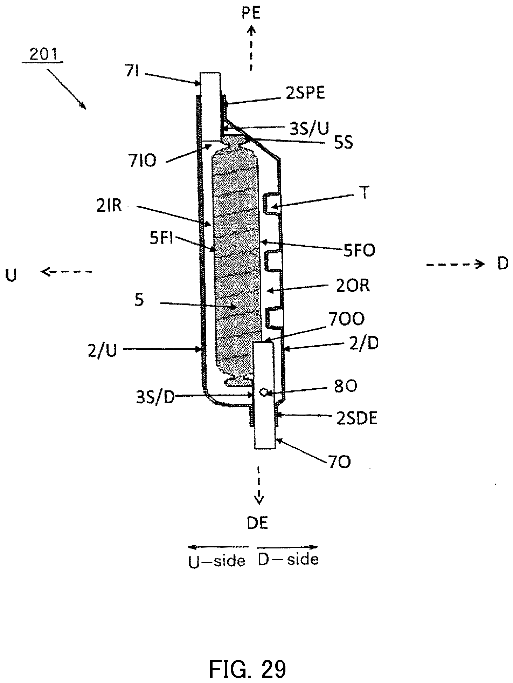

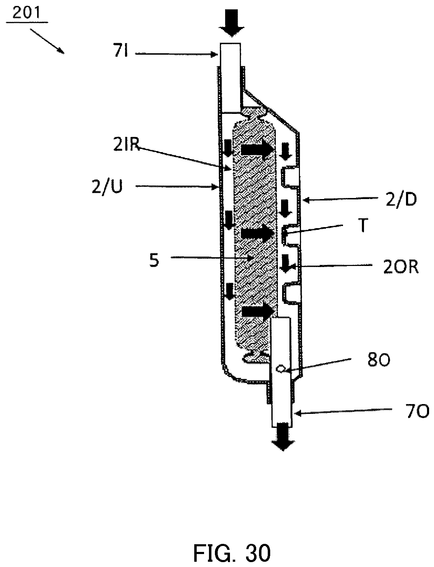

In the actual process of the operation of causing blood to flow in blood processing device 201, because the internal pressure of the blood outflow chamber 2OR side shown in FIGS. 29 and 30 becomes negative, the maximum thickness, which enables the three-dimensional processing, of lower portion-side housing 2/D is set to be 1.5 mm. Furthermore, because the internal pressure of blood inflow chamber 2IR side does not become negative, the lower limit of the aforementioned thickness is set to be 0.8 mm. In a case where the thickness is set to be the value described above, provided that a thickness of upper portion-side housing 2/U is d(U) and a thickness of lower portion-side housing 2/D is d(D), a ratio of d(D)

to d(U) is 100:100 (d(D) is substantially the same as d(U)) to 188:100 (d(D) is substantially two times d(U)). That is, the following relationship is established.

$$d(D)/d(U):[(100/100) \text{ to } (188/100)]$$

Tubular blood inlet section 7I is mounted on one end portion of the upper portion U side on the proximal end PE side of housing 2, and tubular blood outlet section 7O is mounted on one end portion of the lower portion D side on the distal end DE side of housing 2.

Similarly to Types I to III, tubular blood inlet section 7I and blood outlet section 7O are formed of a resin harder than flexible housing 2.

Furthermore, similarly to Types I to III, blood processing member 5 is formed by laminating a plurality of sheets of nonwoven cloth.

On the surface of blood processing member 5 of lower portion-side housing 2/D that faces outer side filtration section 5FO, projections T (referred to as "ribs" in some cases) rising toward the upper portion U side from the lower portion D side are formed. The interior of each of projections T is empty as shown in FIG. 29 or the like, and forms so-called "depression" (referred to as "groove" in some cases) toward the upper portion U side from the lower portion D side.

It is preferable that projections T are formed such that height t thereof becomes about equal to or greater than 3 mm and equal to or smaller than 10 mm.

In a case where height t of projections T is too small and is less than 3 mm for example, when the internal pressure of blood outflow chamber 2OR becomes negative, the adhesion of lower portion-side housing 2/D to outer side filtration section 5FO cannot be sufficiently effectively inhibited.

In contrast, in a case where height t is too large and exceeds 10 mm for example, projections T become too large. Accordingly, it is difficult to form projections T on the interior 2I side of lower portion-side housing 2/D.

Note that projections T can be formed simultaneously with the three-dimensional processing.

Projections T may be formed such that they have an approximate circular shape when seen in the direction of upper portion U (or lower portion D) as illustrated in the drawing. Furthermore, projections T may be formed such that they have an approximate elliptical shape, an approximate polygonal (triangular, quadrangular, pentagonal, or hexagonal) shape, a star shape, and the like when seen in the same direction as described above.

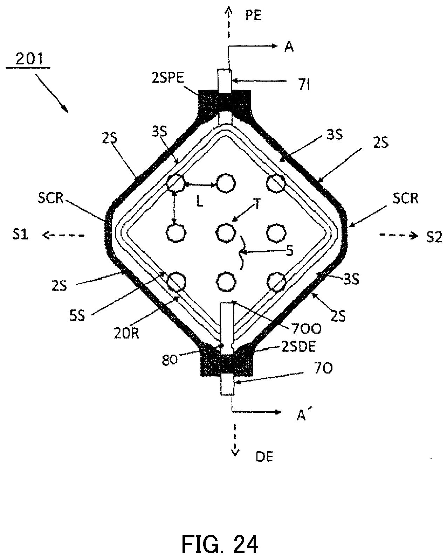

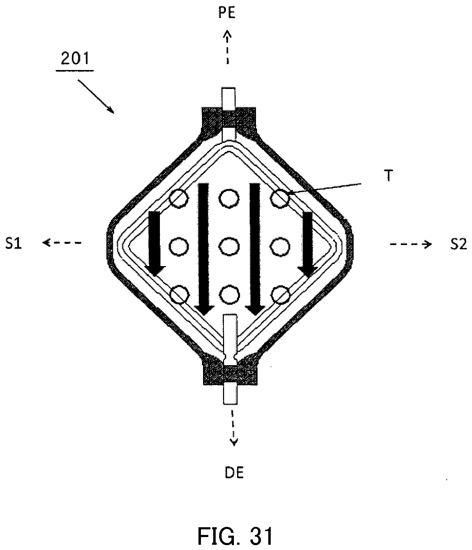

Regarding the arrangement of projections T, as shown in FIG. 24, it is preferable that projections T are regularly arranged approximately in series at certain arrangement intervals in the direction of proximal end PE-distal end DE and in the direction of first side portion SI-second side portion S2. In a case where the projections are arranged in this way, the blood having flowed into blood outflow chamber 2OR as shown in FIG. 31 can rapidly pass through the voids of projections T regularly arranged at certain arrangement intervals and can be rapidly discharged from blood outlet section 7O.

Basically, projections T described above are regularly arranged in the form of a grid. Specifically, arrangement interval L (interval between outer edge portions in the direction of first side portion SI-second side portion S2 and in the direction of proximal end PE-distal end DE) of projections T is preferably set to be about equal to or greater than 20 mm and equal to or smaller than 30 mm.

In a case where interval L between projections T is too small and is less than 20 mm for example, it is difficult to form projections T on the interior 2I side of lower portion-side housing 2/D. Extremely small interval L is not preferable because the flow of blood toward blood outlet section 7O is hindered.

In contrast, in a case where interval L between projections T is too large and exceeds 30 mm for example, the interval between projections T formed on the interior 2I side of lower portion-side housing 2/D becomes too large. Therefore, when the internal pressure of blood outflow chamber 2OR becomes negative, the adhesion of the inner wall of lower portion-side housing 2/D to outer side filtration section 5O cannot be sufficiently effectively inhibited.

The same projections T as those described above can also be formed on the interior 2I side of upper portion-side housing 2/U in the same manner as described above. In a case where projections T are formed on the interior 2I side of upper portion-side housing 2/U, in blood inflow chamber 2IR, a blood flow path can be secured over a large area from the proximal end PE side to the distal end DE side. Therefore, the blood having flowed into blood inflow chamber 2IR from blood inlet 71 can be rapidly filtered through blood processing member 5. Consequently, the filtration time can be further reduced.

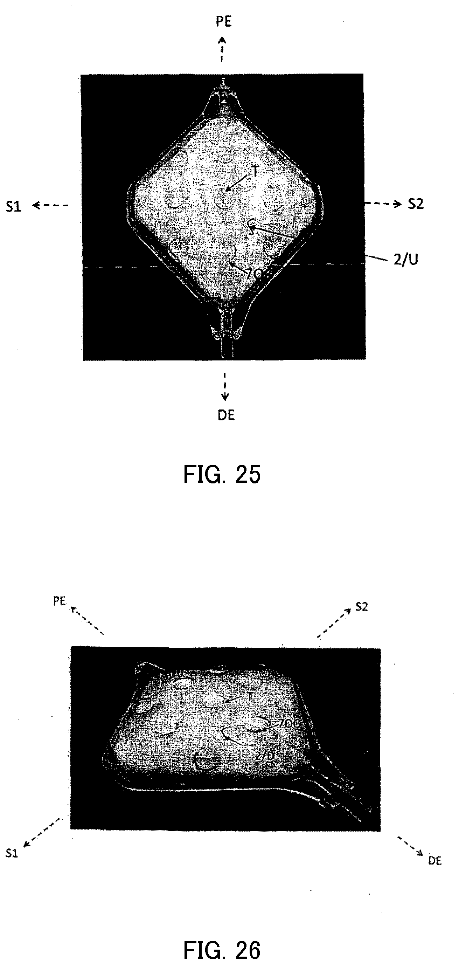

In FIGS. 25 and 31, a total of 9 projections T are arranged including 3 projections T arranged in the direction of proximal end PE-distal end DE and 3 projections T arranged in the direction of first side portion SI-second side portion S2. However, the number of projections T arranged is not limited to this.

A total of 4 to 16 projections T may be arranged including 2 to 4 projections T in the direction of proximal end PE-distal end DE and 2 to 4 projections T in the direction of first side portion S1-second side portion S2.

(Example of assembly process of blood processing device 201 (Type IV))

Hereinafter, an aspect of an embodiment of blood processing device 201 will be described based on an example of an assembly process.

First, blood processing member 5 and outer frame sheet 3 are disposed and fixed as shown in FIG. 28.

More specifically, the outer edge of blood processing member 5 is fixed between the inner edges of upper portion outer frame sheet 3/U and lower portion-side outer frame sheet 3/D.

Similarly to the case of Types I to III, the fixing can be performed by welding by means of external heating by heat sealing or the like, internal heating by a high frequency and an ultrasonic wave, and the like.

Hereinafter, the fixing portions of blood processing member 5 and outer frame sheet 3 described above will be referred to as "sealing portion 5S" in some cases. Furthermore, the non-fixing portions on the inside of blood processing member 5 and outer frame sheet 3 will be referred to as filtration section 5F in some cases. In addition, the surface on the blood inflow chamber 2IR side will be referred to as inner side filtration section 5FI, and the surface on the blood outflow chamber 2OR side will be referred to as outer side filtration section 5FO in some cases.

The outer edges of the upper portions-side outer frame sheet 3/U and outer frame sheet 3/D on the lower portion D side are fixed as well. Hereinafter, the fixing portions will be referred to as "outer frame sealing portion 3S (3S/U and 3S/D) in some cases".

Upper portion-side lengthened section 3L/U is formed on the proximal end PE side of upper portion-side outer frame sheet 3/U, and lower portion-side lengthened section 3L/D is formed on the distal end DE side of lower portion-side outer frame sheet 3/U.

Blood inlet section 71 is fixed between upper portion-side lengthened section 3L/U and upper portion-side housing 2/U. Furthermore, blood outlet section 7O is fixed between lower portion-side lengthened section 3L/D and lower portion-side housing 2/D (see FIG. 28).

Blood processing member 5 is further compressed in the fixing portion (sealing portion 5S) of blood processing member 5 and outer frame sheet 3 than in the non-fixing portion (filtration section 5F) on the inside of sealing portion 5S.

Then, from the upper portion U side to the lower portion D side, the following members are disposed. In the proximal end PE-side area, upper portion-side housing 2/U, blood inlet section 71, upper portion-side lengthened section 3L/U, and lower portion-side housing 2/D are disposed in this order. Furthermore, in the distal end DE-side area, upper portion-side housing 2/U, lower portion-side lengthened section 3L/D, blood outlet section 7O, and lower portion-side housing 2/U are disposed in this order.

Thereafter, corners CR/S1 and CR/S2 on the first side portion S1 side and the second side portion S2 side are fixed by heating sealing (hereinafter, the fixing portion will be referred to as "corner sealing portion SCR" in some cases).

Subsequently, upper portion-side housing 2/U, blood inlet section 71, upper portion-side lengthened section 3L/U, and lower portion-side housing 2/D in proximal end PE-side area, upper portion-side housing 2/U, lower portion-side lengthened section 3L/D, blood outlet section 7O, and lower portion-side housing 2/U in the distal end DE-side area, and peripheries (edge portions and vicinities) of these are fixed by heat sealing.

Hereinafter, the fixing portions will be referred to as "proximal end-side sealing portion 2SPE and distal end-side sealing portion 2SDE" in some cases (see FIGS. 23 to 25).

The proximal end PE side and the distal end DE side may be simultaneously fixed. Alternatively, the proximal end PE side may be fixed first, and then the distal end DE side may be fixed. As another option, the distal end DE side may be fixed first, and then the proximal end PE side may be fixed.

Then, outer frame sealing portions 3S/U and 3S/D of the outer edges of 4 sides (oblique sides) of blood processing member 5 are fixed to upper portion-side housing 2/U and lower portion-side housing 2/D by heating sealing or the like. Hereinafter, the fixing portions will be referred to as "sealing portion 2S" in some cases. In the manner described so far, blood processing device 201 in FIG. 29 is completed.

(Form of blood processing device 201)

The form of blood processing device 201 will be described.

For example, as shown in FIG. 29, in the proximal end PE side, at the site on which blood inlet section 71 is mounted, from the upper portion U side to the lower portion D side, upper portion-side housing 2/U, blood inlet section 71, upper portion-side lengthened section 3L/U, and lower portion-side housing 2/D are fixed in this order.

The proximal end PE-side area of blood processing member 5 is fixed to upper portion-side lengthened section 3L/U through outer frame sealing portion 3S/U of outer frame sheet 3/U on the upper portion side.

In the distal end DE-side area, at the site on which blood outlet section 7O is mounted, from the upper portion U side to the lower portion D side, upper portion-side housing 2/U, lower portion-side lengthened section 3L/D, blood outlet section 7O, and lower portion-side housing 2/D are fixed in this order.

The distal end DE-side area of blood processing member 5 is fixed to lower portion-side lengthened section 3L/D through outer frame sealing portion 3S/D of the lower portion-side outer frame sheet 3/D.

The distal end DE-side area of blood inlet section 71 is stuck into the interior of blood inflow chamber 2IR of housing 2. In blood outlet section 7O, outlet side hole 8O is formed in the vicinity of the fixing portion (on the distal end-side sealing portion 2S/DE side). Outlet side hole 8O may be formed at 2 sites or formed at 3 to 4 sites. In a case where outlet side hole 8O is formed, blood can rapidly flow out from the distal end DE side of blood outflow chamber 2OR.

(Internal structure of housing 2)

The internal structure of housing 2 will be specifically described.

As shown in FIGS. 29 and 30, the space between the inner periphery of upper portion-side housing 2/U and inner side filtration section 5FI of blood processing member 5 becomes blood inflow chamber 2IR.

Likewise, as shown in FIGS. 29 and 30, the space between the inner periphery of lower portion-side housing 2/D and outer side filtration section 5FO of blood processing member 5 becomes blood outflow chamber 2OR.

Blood inlet section 7I communicates with blood inflow chamber 2IR, and blood outlet section 7O communicates with blood outflow chamber 2OR.

A plurality of projections T described above are formed on the inside of lower portion-side housing 2/D.

(Flow of blood in blood processing device 201 (Type IV))

Next, the flow of blood in blood processing device 201 (Type IV) will be described.

As illustrated in FIGS. 30 and 31, blood from the proximal end PE side of blood inlet section 7I passes through inlet opening 7IO on the distal end DE side and flows into blood inflow chamber 2IR.

The blood in blood inflow chamber 2IR passes through inner side filtration section 5FI and then through outer side filtration section 5FO and moves into blood outflow chamber 2OR.

Even though the internal pressure of blood outflow chamber 2OR becomes negative, the inner wall of lower portion-side housing 2/D is deformed to a small extent in a direction along which the inner wall contacts the outer side filtration section 5OF side of blood processing member 5. Furthermore, even though the inner wall is deformed in the direction along which the inner wall contacts the outer side filtration section 5OF side, due to projections T, protruding toward the outer side filtration section 5OF side, on the inner wall of lower portion-side housing 2/D, the inner wall is hindered from contacting the outer side filtration section 5OF side.

As a result, a sufficient blood flow path can be secured in blood outflow chamber 2OR. In addition, the blood flow path in the vicinity of outlet opening 7OO on the proximal end PE side of blood outlet section 7O is not narrowed or blocked. Accordingly, the filtration time can be significantly reduced.

Due to outlet side hole 8 (formed in the vicinity of distal end-side sealing portion 2SDE) of blood outlet section 7O, the blood can more rapidly flow out, and the filtration time can be further reduced.

The blood processing member 5 of blood processing device 201 of embodiment 4 (Type IV) is the same as blood processing member 5 of blood processing devices 1 and 1' of embodiment 1 (Type I) and embodiment 2 (Type II), in terms of the constituent material, the material, the fiber diameter, the number of sheets to be laminated, the thickness, the form of lamination, the lamination direction, the lamination order, the material of housing 2 and outer frame sheet 3, and the material of blood inlet section 71 and blood outlet section 7O.

Hereinafter, based on examples, specific embodiments of blood processing device 1 (Type I), blood processing device 1' (Type II), and blood processing device 101 (Type III) will be described.

[Example B]

In order to compare blood processing device 1 (Type I) of embodiment 1, blood processing device 1' (Type II), and blood processing device 101 (Type III) with each other in terms of the filtration performance, a filtration performance test was performed.

(Example 2) (Type I)

As blood processing device 1 (Type I) of embodiment 1, a device assembled in the same manner as in Example 1 was used.

As blood for a filtration test, bovine blood was used as in Example 1, and a filtration test was performed in the same manner as in Example 1. As a result, a time $\theta$ taken for the filtration of 400 mL of blood to be finished was 16.0 minutes.

(Example 3) (Type II)

Example 3 was a test in which blood processing device 1' (Type II) of embodiment 2 was used. In Example 3, by using bovine blood, a filtration test was performed in the same manner as in Example 1, except that blood processing device 1 (Example 1) [assembled as described in FIGS. 3 to 8] of the embodiment 1 was flipped 180° such that blood inlet section 7I (7I') and blood outlet section 7O (7O') were used by being switched with each other.

A time $\theta$ taken for the filtration of 400 mL of blood to be finished was 18.0 minutes.

(Example 4) (Type III)

Example 4 was a test performed using blood processing device 101 (Type III) of embodiment 3. In Example 4, a device assembled as below according to the description in FIGS. 15 to 20 was used.

Five sheets (n = 5) of nonwoven cloth (thickness d = 120 to 130 $\mu$m, fiber diameter l = 5 to 10.0 $\mu$m, length L of one side = 72 mm $\times$ 72 mm) made of polyethylene terephthalate were laminated and used as each of a pair of (two) first blood processing members (5/PEU and 5/PED) in the proximal end-side area (PE-side Area) in blood processing device 101 (total thickness Td = 600 to 650 $\mu$m).

Furthermore, 30 sheets (n = 30) of nonwoven cloth (thickness d = 120 to 130 $\mu$m, fiber diameter l = 1 to 4 $\mu$m, length L of one side = 72 mm $\times$ 72 mm) made of polyethylene terephthalate were laminated and used as each of a pair of (two) second blood processing members (5/DEU and 5/DED) in the distal end-side area (DE-side Area) (total thickness Td = 3.6 to 3.9 mm).

Each of the members was fixed by means of heat sealing by using outer frame sheet 3 and a high-frequency welding machine.

Blood processing device 101 (Type III) was prepared as below. A pair of (two) first blood processing members (5/PEU and 5/PED) in the proximal end-side area (PE-side Area) and a pair of (two) second blood processing members (5/DEU and 5/DED) in the distal end-side area (DE-side Area) were fixed to outer frame sheet 3 made of soft polyvinyl chloride, and outer frame sheet 3 was fixed to flexible housing 2 made of polyvinyl chloride. In the interior of the pair of proximal end-side first blood processing members 5/PE and the pair of distal end-side second blood processing members 5/DE disposed to face each other, blood inflow chambers (2IR/PE and 2IR/DE) were formed. Furthermore, between the exterior (outside) of the pair of blood processing members (5/PE and 5/DE) and flexible housings (2/U and 2/D), blood outflow chambers (2OR/PE and 2OR/DE) were formed.

As shown in FIG. 14, the interior of flexible housings (2/U and 2/D) was divided into blood inflow chamber 2/IR and blood outflow chamber 2/OR across blood processing members (5/PE and 5/DE). Blood inlet section 7I was mounted on the proximal end side of first blood inflow chamber 2IR/PE, and blood outlet section 7O was mounted on the distal end side of second blood inflow chamber 2IR/DE. All of the fixing and the mounting described above were performed by means of heat sealing by using a high-frequency welding machine.

As blood for a filtration test, bovine blood was used as in Example 1, and the filtration test was performed in the same manner as in Example 1. As a result, a time $\theta$ taken for the filtration of 400 mL of blood to be finished was 15.7 minutes.

(Results and study)

At first, it was assumed that Example 2 (Type I) to Example 4 (Type III) will be listed as Example 4, Example 3, and Example 2 in order of shortest to longest filtration time $\theta$ (rapidity of filtration). However, the results showed that the examples were listed as Example 4 ($\theta$ = 15.7 minutes), Example 2 ($\theta$ = 16.0 minutes), and Example 3 ($\theta$ = 18 minutes) in order of shortest to longest filtration time $\theta$ (rapidity of filtration). It is assumed that in a case where the constitution (number of sheets and the like) of first blood processing members (5/PEU and 5/PED) of the proximal end-side area (PE-side Area) and second blood processing members (5/DEU and 5/DED) of the distal end-side area (DE-side Area) in Example 4 (blood processing device 101) (Type III) is further studied, the filtration time θ could be further reduced (shortened).

Theoretically, Example 3 (Type II) is assumed to be more advantageous in blood filtration compared to Example 2 (Type I). Therefore, in a case where a measure to inhibit the expansion of (flexible) housing 2 (for example, improving the hardness of the housing and the like) is taken in Example 3 (Type II), the filtration time θ is expected to be further reduced than in Example 2 (Type I).

Herein, for example, similarly to blood processing device 201 (Type IV) of embodiment 4 described above, in blood processing devices 1, 1', and 101, improving the hardness of the housing and the like is achieved by applying means such as (a) forming a thick housing, (b), three-dimensionally forming a housing such that interior (2I) thereof rises from outer edge portion (2F), or (c) providing a plurality of projections (T) in interior (2I) of lower portion (D-side) second housing (2/D) or in interior (2I) of second housing (2/D) and upper portion (U-side) first housing (2/U) to upper portion (U-side) first housing (2/U) and lower portion (D-side) second housing (2/D).

Hereinafter, specific embodiments of blood processing device 201 (Type IV) will be described based on examples.

[Example C]

(Example 5) (Type IV)

As a blood processing device of Example 5, blood processing device 201 (Type IV) assembled as below was used.

Specifically, 60 sheets (n = 60) of nonwoven cloth (thickness d = 130 $\mu$m, fiber diameter l = 1.8 $\mu$m, length Ls of one side = 66 mm × 66 mm) made of polyethylene terephthalate were laminated and used as blood processing member 5 (total thickness Td of the processing member = 7.8 mm).

Upper portion-side housing 2/U was prepared by three-dimensionally processing a flexible sheet having a thickness of 0.8 mm, and lower portion-side housing 2/D was prepared by three-dimensionally processing a flexible sheet having a thickness of 1.0 mm. Projections T of lower portion-side housing 2/D were formed simultaneously with the three-dimensional processing. That is, as shown in FIG. 25, the formed projections T had a height (t) of 5 mm and had a circular shape, the number (N) of projections T arranged was 9, and the arrangement interval (L) thereof was 25 mm. In the upper portion-side housing 2/U, projections T having the same height and shape as those described above were formed, and the number of projections T arranged was the same as described above.

Blood processing device 201 (Type IV) was prepared as below (see FIG. 28). Blood processing member 5 was fixed to outer frame sheet 3 made of soft polyvinyl chloride, and outer frame sheet 3 was fixed to a pair of upper and lower flexible housings (2/U and 2/D) made of polyvinyl chloride.

The interior of flexible housings (2/U and 2/D) was divided into blood inflow chamber 2IR and blood outflow chamber 2OR across blood processing member 5. Blood inlet section 71 was mounted on the proximal end side of blood inflow chamber 2IR, and blood outlet section 7O was mounted on the distal end side of blood outflow chamber 2OR.

The fixing of the members was performed by means of heat sealing by using a high-frequency welding machine.

As blood for a filtration test, bovine blood was used as in Example 1, and the filtration test was performed in the same manner as in Example 1. As a result, a time θ taken for the filtration of 400 mL of blood to be finished was 29.5 minutes.

(Comparative Example 2)

As a blood processing device of Comparative Example 2, a device which was prepared in the same manner as in Comparative Example 1 and had the same form as the blood processing device of Comparative Example 1 was used.

Note that a flexible sheet having a thickness 0.4 mm was directly used as upper portion-side housing 2/U and lower portion-side housing 2/D without being three-dimensionally processed.

As blood for a filtration test, bovine blood prepared in the same manner as in Example 1 was used, and the same test as that in Example 1 was performed. As a result, a time θ taken for the filtration of 400 mL of blood to be finished was 37 minutes.

(Study of result)

The results of Example 5 and Comparative Example 2 were studied by comparison. In Comparative Example 2, the time θ taken for the blood filtration to be finished was 37 minutes. However, in Example 5, the time θ taken for the blood filtration to be finished was only 29.5 minutes. From this result, it was confirmed that Example 5, which is a specific embodiment of blood processing device 201 (Type IV), can secure a relatively wider blood flow path in blood outflow chamber 2OR compared to comparative examples even though the internal pressure of blood outflow chamber 2OR becomes negative at the time of blood filtration, and can reduce the blood filtration time.

Reference Signs List

1, 1', 101, 201 Blood processing device
2 (Flexible) housing

2/U Upper portion-side (first) housing
2/D Lower portion-side (second) housing
SH Flexible sheet
SH/U Upper portion-side (first) flexible sheet
SH/D Lower portion-side (second) flexible sheet
2I Interior (area)
2F Outer edge portion
2.1 (Inner side) first blood chamber
2.2 (Outer side) second blood chamber
2IR Blood inflow chamber
2IR/U, 2IR'/U Upper portion-side blood inflow chamber
2IR/D, 2IR'/D Lower portion-side blood inflow chamber
2IR/PE Proximal end-side (first) blood inflow chamber
2IR/DE Distal end-side (second) blood inflow chamber
2OR, 2OR' Blood outflow chamber
2OR/U Upper portion-side blood outflow chamber
2OR/D Lower portion-side blood outflow chamber
2OR/PE Proximal end-side (first) blood outflow chamber
2OR/DE Distal end-side (second) blood outflow chamber
2OR/PEU Proximal end side-upper portion side (first) blood outflow chamber
2OR/PED Proximal end side-lower portion side (first) blood outflow chamber
2OR/PES Proximal end side-side portion side (first) blood outflow chamber
2OR/DEU Distal end side-upper portion side (second) blood outflow chamber
2OR/DED Distal end side-lower portion side (second) blood outflow chamber
2OR/DES Distal end side-side portion side (second) blood outflow chamber
2S Housing sealing portion (oblique side sealing portion)
2SPE Proximal end-side sealing portion
2SDE Distal end-side sealing portion
3 Outer frame sheet
3/U Upper portion-side (first) outer frame sheet
3/D Lower portion-side (second) outer frame sheet
3/UU Upper (first of the first) outer frame sheet on upper portion side
3/UD Lower (second of the first) outer frame sheet on upper portion side
3/DU Upper (first of the second) outer frame sheet on lower portion side
3/DD Lower (second of the second) outer frame sheet on lower portion side
3/PE Proximal end-side (first) outer frame sheet
3/DE Distal end-side (second) outer frame sheet
3/PEU Proximal end side-upper portion side (first of the first) outer frame sheet
3/PED Proximal end side-lower portion side (second of the first) outer frame sheet

3/DEU Distal end side-upper portion side (first of the second) outer frame sheet
3/DED Distal end side-lower portion side (second of the second) outer frame sheet
3/PEUU Upper (first of the first) outer frame sheet on proximal end side-upper portion side
3/PEUD Lower (first of the first) outer frame sheet on proximal end side-upper portion side
3/3/PEDU Upper (second of the first) outer frame sheet on proximal end side-lower portion side
3/PEDD Lower (second of the first) outer frame sheet on proximal end side-lower portion side
3/DEUU Upper (first of the second) outer frame sheet on distal end side-upper portion side
3/DEUD Lower (first of the second) outer frame sheet on distal end side-upper portion side
3/DEDU Upper (second of the second) outer frame sheet on distal end side-lower portion side
3/DEDD Lower (second of the second) outer frame sheet on distal end side-lower portion side
3S Outer frame sealing portion
3S/U Upper portion-side (first) outer frame sealing portion
3S/D Lower portion-side (second) outer frame sealing portion
3L Lengthened section
3L/U Upper portion-side (first) lengthened section
3L/D Lower portion-side (second) lengthened section
3L/PE Proximal end-side (first) lengthened section
3L/DE Distal end-side (second) lengthened section
3L/PEU Proximal end side-upper portion side (first) lengthened section
3L/PED Proximal end side-lower portion side (first) lengthened section
3L/DEU Distal end side-upper portion side (second) lengthened section
3L/DED Distal end side-lower portion side (second) lengthened section
5 blood processing member
5/U Upper portion-side (first) blood processing member
5/D Lower portion-side (second) blood processing member
5/PEU, 5/DEU Upper portion-side (first) blood processing member
5/PED, 5/DED Lower portion-side (second) blood processing member
5/PE Proximal end-side (first) blood processing member
5/DE Proximal end-side (second) blood processing member
5/PEU Proximal end side-upper portion side (first) blood processing member
5/PED Proximal end side-lower portion side (first) blood processing member
5/DEU Distal end side-upper portion side (second) blood processing member

5/DED Distal end side-lower portion side (second) blood processing member

5S Sealing portion

5F Filtration section

5FI, 5FI' Inner side (interior) filtration section

5FI/U, 5FI'/U Upper portion-side (first) inner side filtration section

5FI/D, 5FI'/D Lower portion-side (second) inner side filtration section

5FI/PE Proximal end-side (first) inner side filtration section

5FI/DE Distal end-side (second) inner side filtration section

5FI/PEU Proximal end side-upper portion side (first) inner side filtration section

5FI/PED Proximal end side-lower portion side (first) inner side filtration section

5FI/DEU Distal end side-upper portion side (second) inner side filtration section

5FI/DED Distal end side-lower portion side (second) inner side filtration section

5FO, 5FO' Outer side (exterior) filtration section

5FO/U, 5FO'/U Upper portion-side (first) outer side filtration section

5FO/D, 5FO'/D Lower portion-side (second) outer side filtration section

5FO/PE Proximal end-side (first) outer side filtration section

5FO/DE Distal end-side (second) outer side filtration section

5FO/PEU Proximal end side-upper portion side (first) outer side filtration section

5FO/PED Proximal end side-lower portion side (first) outer side filtration section

5FO/DEU Distal end side-upper portion side (second) outer side filtration section

5FO/DED Distal end side-lower portion side (second) outer side filtration section

7I, 7I' Blood inlet section

7IO, 7IO' Inlet opening

7O, 7O' Blood outlet section

7OO Outlet opening

8 Side hole

8I Inlet side hole

8O Outlet side hole

CR Corner

SCR Corner sealing portion

DE Distal end

PE Proximal end

S1 First side portion

S2 Second side portion

U Upper portion

D Lower portion

CR Corner

T Projection

t Height of projection

L Arrangement interval of projections

**Claims**

1. A blood processing device (1, 1', 101), comprising:

   a housing (2);
   a blood inlet section (7I, 7I') disposed on a proximal end (PE) side of the housing (2);
   a blood outlet section (7O, 7O') disposed on a distal end (DE) side of the housing (2);
   an upper portion-side first blood processing member [5/U, (5/PEU, 5/DEU)] which is disposed in the housing (2) and formed by laminating a plurality of sheets of nonwoven cloth having a predetermined thickness; and
   a lower portion-side second blood processing member [5/D, (5/PED, 5/DED)] which is disposed in the housing (2) and formed by laminating a plurality of sheets of nonwoven cloth having a predetermined thickness,
   wherein the upper portion-side first blood processing member [5/U, (5/PEU, 5/DEU)] and the lower portion-side second blood processing member [5/D, (5/PED, 5/DED)] each have an inner side filtration section (5FI, 5FI') and an outer side filtration section (5FO, 5FO'), wherein the upper portion-side first blood processing member [5/U, (5/PEU, 5/DEU)] and the lower portion-side second blood processing member [5/D, (5/PED, 5/DED)] are disposed such that the inner side filtration sections (5FI, 5FI') thereof face each other,
   an inner side first blood chamber (2.1) is formed between the inner side filtration sections (5FI, 5FI'), and
   an outer side second blood chamber (2.2) is formed between an inner periphery of the housing (2) and the outer side filtration sections (5FO, 5FO') of the upper portion-side first blood processing member [5/U, (5/PEU, 5/DEU)] and the lower portion-side second blood processing member [5/D, (5/PED, 5/DED)].

2. The blood processing device (1, 1', 101) according to claim 1,
   wherein the inner side first blood chamber (2.1) functions as a blood inflow chamber (2IR) or a blood outflow chamber (2OR'), and
   the outer side second blood chamber (2.2) functions as a blood outflow chamber (2OR) or a blood inflow chamber (2IR').

3. The blood processing device (1, 1', 101) according to claim 1,
   wherein an upper portion-side first housing (2/U) and a lower portion-side second housing (2/D) are formed by processing a thick flexible sheet into a three-dimensional shape,
   the upper portion-side first housing (2/U) and the lower portion-side second housing (2/D) are three-dimensionally formed such that an interior (2I) thereof rises from an outer edge portion (2F), and a plurality of projections (T) are provided in the interior (2I) of the second housing (2/D) or in the interior (2I) of the second housing (2/D) and the first housing (2/U).

**4.** The blood processing device (1, 1') according to claim 1 or 2,
wherein the blood inlet section (71, 71') is disposed on the proximal end (PE) side of the upper portion-side first housing (2/U) and the lower portion-side second housing (2/D),
the blood outlet section (7O, 7O') is disposed on the distal end (DE) side of the first housing (2/U) and the second housing (2/D), and

(i) in a case where the blood inlet section (71) is formed to communicate with the inner side first blood chamber (2.1), the blood outlet section (7O) is formed to communicate with the outer side second blood chamber (2.2), the inner side first blood chamber (2.1) functions as a blood inflow chamber (2IR/U, 2IR/D), and the outer side second blood chamber (2.2) functions as a blood outflow chamber (20R/U, 2OR/D), or
(ii) in a case where the blood inlet section (7I') is formed to communicate with the outer side second blood chamber (2.2), the blood outlet section (7O') is formed to communicate with the inner side first blood chamber (2.1), the outer side second blood chamber (2.2) functions as a blood inflow chamber (2IR'/U, 2IR'/D), and the inner side first blood chamber (2.1) functions as a blood outflow chamber (2OR').

**5.** The blood processing device (1, 1') according to claim 4,
wherein an outer edge of the first blood processing member (5/U) on an upper portion side is fixed to an inner edge of a pair of upper and lower first outer frame sheets (3/UU, 3/UD) on an upper portion side in a state where the outer edge is held by the first outer frame sheets (3/UU, 3/UD),
an inner edge of the first flexible housing (2/U) is fixed to an outer edge of the first outer frame sheets (3/UU, 3/UD),
an outer edge of the second blood processing member (5D) on a lower portion side is fixed to an inner edge of a pair of upper and lower second outer frame sheets (3/DU, 3/DD) on a lower portion side in a state where the outer edge is held by the second outer frame sheets (3/DU and 3/DD), and
an inner edge of the second flexible housing (2/D) is fixed to an outer edge of the second outer frame sheets (3/DU, 3/DD).

**6.** The blood processing device (1, 1') according to claim 5,
wherein in the pair of upper and lower first outer frame sheets (3/UU, 3/UD) on the upper portion side and the pair of upper and lower second outer frame sheets (3/DU, 3/DD) on the lower portion side, an upper portion-side first lengthened section (3L/U) and a lower portion-side second lengthened section (3L/D) are formed on the proximal end (PE) side or the distal end (DE) side, and

(i) on the proximal end (PE) side where the blood inlet section (71) is fixed, from an upper portion (U) side to a lower portion (D) side, the upper portion-side first housing (2/U), the upper portion-side first lengthened section (3L/U), the blood inlet section (71), the lower portion-side second lengthened section (3L/D), and the lower portion-side second housing (2/D) are fixed in this order, and
on the distal end (DE) side where the blood outlet section (7O) is fixed, from the upper portion (U) side to the lower portion (D) side, the upper portion-side first housing (2/U), the blood outlet section (7O), and the lower portion-side second housing (2/D) are fixed in this order, or
(ii) on the proximal end (PE) side where the blood inlet section (7I') is fixed, from the upper portion (U) side to the lower portion (D) side, the upper portion-side first housing (2/U), the blood inlet section (7I'), and the lower portion-side second housing (2/D) are fixed in this order, and
on the distal end (DE) side where the blood outlet section (7O') is fixed, from the upper portion (U) side to the lower portion (D) side, the upper portion-side first lengthened section (3L/U), the upper portion-side first housing (2/U), the blood outlet section (7O'), the lower portion-side second lengthened section (3L/D), and the lower portion-side second housing (2/D) are fixed in this order.

**7.** The blood processing device (1, 1') according to claim 4,
wherein an inlet side hole (8I) is formed at a position closer to the proximal end (PE) side than an inlet opening (7IO) on an extreme distal end (DE) side of the blood inlet section (7I), and
the inlet side hole (8I) is formed in the vicinity of the proximal end (PE) side of an inner side filtration section (5FI/U, 5FI/D) of the blood processing member (5/U, 5/D), or
an outlet side hole (8O) is formed at a position closer to the distal end (DE) side than an outlet opening (7OO) on an extreme proximal end (PE) side of the blood outlet section (7O'), and
the outlet side hole (8O) is formed in the vicinity of the distal end (DE) side of an inner side filtration section (5FI'/U, 5FI'/D) of the blood processing member (5/U, 5/D).

8. The blood processing device (101) according to claim 1,
wherein a pair of upper and lower proximal end-side first blood processing members (5/PEU, PED) formed by laminating a plurality of sheets of nonwoven cloth having a predetermined thickness are disposed in a proximal end (PE) side (PE-side Area) in the housing (2), and
a pair of upper and lower distal end-side second blood processing members (5/DEU, DED) formed by laminating a plurality of sheets of nonwoven cloth having a predetermined thickness are disposed in a distal end (DE) side (DE-side Area) in the housing (2), wherein
the pair of upper and lower proximal end-side first blood processing members (5/PEU, PED) and the pair of upper and lower distal end-side second blood processing members (5/DEU, DED) each have the inner side filtration section (5FI) and the outer side filtration section (5FO), wherein
the pair of upper and lower proximal end-side first blood processing members (5/PEU, PED) and the pair of upper and lower distal end-side second blood processing members (5/DEU, DED) are disposed such that the inner side filtration sections (5FI) thereof face each other,
a proximal end-side first blood inflow chamber (2IR/PE) is formed between the inner side filtration sections (5FI), which face each other, of the pair of upper and lower proximal end-side first blood processing members (5/PEU, PED),
a distal end-side second blood inflow chamber (2IR/DE) is formed between the inner side filtration sections (5FI), which face each other, of the pair of upper and lower distal end-side second blood processing members (5/DEU, DED),
a proximal end-side blood outflow chamber (2OR/PE) and a distal end-side blood outflow chamber (2OR/DE) are formed between the inner periphery of the housing (2) and the outer side filtration sections (5FO) of the pair of upper and lower proximal end-side first blood processing members (5/PEU, PED) and the pair of upper and lower distal end-side second blood processing members (5/DEU, DED),
the blood inlet section (71) communicates with the proximal end-side first blood inflow chamber (2IR/PE), and
the blood outlet section (7O) communicates with the distal end-side second blood inflow chamber (2IR/DE).

9. The blood processing device (101) according to claim 8,
wherein an outer edge of each of the pair of upper and lower proximal end-side first blood processing members (5/PEU, 5/PED) is fixed to an inner edge of a pair of upper and lower proximal end-side first outer frame sheets (3/PEU, 3/PED) in a state where the outer edge is held by the pair of upper and lower proximal end-side first outer frame sheets (3/PEU, 3/PED), and
an outer edge of the pair of upper and lower proximal end-side first outer frame sheets (3/PEU, 3/PED) is fixed to the housing (2), and
an outer edge of each of the pair of upper and lower distal end-side second blood processing members (5/DEU, 5/DED) is fixed to an inner edge of a pair of upper and lower distal end-side second outer frame sheets (3/DEU, 3/DED) in a state where the outer edge is held by the pair of upper and lower distal end-side second outer frame sheets (3/DEU, 3/DED), and
an outer edge of the pair of upper and lower distal end-side second outer frame sheets (3/DEU, 3/DED) is fixed to the housing (2).

10. The blood processing device (101) according to claim 9,
wherein a pair of upper and lower first lengthened sections (3L/PEU, 3L/PED) are fixed to the proximal end (PE) side of the pair of upper and lower proximal end-side first outer frame sheets (3/PEU, 3/PED),
a pair of upper and lower second lengthened sections (3L/DEU, 3L/DED) are fixed to the distal end (DE) side of the pair of upper and lower distal end-side second outer frame sheets (3/DEU, 3/DED),
on the proximal end (PE) side where the blood inlet section (71) is fixed, from the upper portion (U) side to the lower portion (D) side, the first housing (2/U), the first lengthened section (3L/PEU), the blood inlet section (71), the first lengthened section (3L/PED), and the second housing (2/D) are fixed in this order, and
on the distal end (DE) side where the blood outlet section (7O) is fixed, from the upper portion (U) side to the lower portion (D) side, the first housing (2/U), the second lengthened section (3L/DEU), the blood outlet section (7O), the second lengthened section (3L/DED), and the second housing (2/D) are fixed in this order.

11. The blood processing device (101) according to claim 8,
wherein an inlet side hole (8I) is formed at a position closer to the proximal end (PE) side than an inlet opening (71O) on an extreme distal end (DE) side of the blood inlet section (71), and
the inlet side hole (8I) is formed in the vicinity of the proximal end (PE) side of an inner side filtration section (5FI/PE) of the proximal end-side first blood processing member (5/PEU, 5/PED), and
an outlet side hole (8O) is formed at a position closer to the distal end (DE) side than an outlet opening (7OO) on an extreme proximal end (PE) side of the blood outlet section (7O), and
the outlet side hole (8O) is formed in the vicinity of the distal end (DE) side of an inner side filtration section (5FI/DE) of the distal end-side second blood processing member (5/DEU, 5/DED).

12. A blood processing device (201), comprising:

an upper portion-side first housing (2/U) and a lower portion-side second housing (2/D) formed of a thick flexible sheet processed into a three-dimensional shape;
a blood inlet section (71) disposed on a proximal end (PE) side of the upper portion-side first housing (2/U) and the lower portion-side second housing (2/D);
a blood outlet section (7O) disposed on a distal end (DE) side of the upper portion-side first housing (2/U) and the lower portion-side second housing (2/D); and
a blood processing member (5) which is disposed in the upper portion-side first housing (2/U) and the lower portion-side second housing (2/D) and formed by laminating a plurality of sheets of nonwoven cloth having a predetermined thickness,
wherein the blood processing member (5) has an inner side filtration section (5FI) and an outer side filtration section (5FO),
a blood inflow chamber (2IR) is formed between the inner periphery of the upper portion-side first housing (2/U) and the inner side filtration section (5FI) of the blood processing member (5),
a blood outflow chamber (2OR) is formed between the inner periphery of the lower portion-side second housing (2/D) and the outer side filtration section (5FO) of the blood processing member (5),
the blood inlet section (71) communicates with the blood inflow chamber (2IR),
the blood outlet section (7O) communicates with the blood outflow chamber (2OR),
the first housing (2/U) and the second housing (2/D) are three-dimensionally formed such that an interior (2I) thereof rises from an outer edge portion (2F), and
a plurality of projections (T) are provided in the interior (2I) of the second housing (2/D) or in the interior (2I) of the second housing (2/D) and the first housing (2/U).

13. The blood processing device (201) according to claim 12,
wherein the first housing (2/U) and the second housing (2/D) are formed by processing a flexible sheet having a thickness equal to or greater than 0.8 mm and equal to or smaller than 1.5 mm into a three-dimensional shape, wherein
provided that a thickness of the first housing (2/U) is [d(U)] and a thickness of the second housing (2/D) is [d(D)], a ratio of [d(D)] to [d(U)] is 100:100 to 188:100.

14. The blood processing device (201) according to claim 13,
wherein the projections (T) have a height equal to or greater than 3 mm and equal to or smaller than 10 mm, and
an arrangement interval between the projections (T) is equal to or greater than 20 mm and equal to or smaller than 30 mm.

15. The blood processing device (201) according to claim 12,
wherein in the blood outlet section (7O), an outlet side hole (8O) is formed in the vicinity of a distal end-side sealing portion (2SDE).

FIG. 1

FIG. 2

FIG. 3

FIG. 4

FIG. 5

FIG. 6

FIG. 7

FIG. 8

FIG. 9

FIG. 10

FIG. 11

FIG. 12B

FIG. 12A

FIG. 13

FIG. 14

FIG. 15

FIG. 16

FIG. 17

FIG. 18

FIG. 19

FIG. 20

FIG. 21

FIG. 22

FIG. 23

FIG. 24

FIG. 25

FIG. 26

FIG. 27

FIG. 28

FIG. 29

FIG. 30

FIG. 31

## INTERNATIONAL SEARCH REPORT

| | International application No. |
|---|---|
| | PCT/JP2017/026482 |

**A. CLASSIFICATION OF SUBJECT MATTER**
*A61M1/02(2006.01)i*

According to International Patent Classification (IPC) or to both national classification and IPC

**B. FIELDS SEARCHED**

Minimum documentation searched (classification system followed by classification symbols)
A61M1/02

Documentation searched other than minimum documentation to the extent that such documents are included in the fields searched

| Jitsuyo Shinan Koho | 1922-1996 | Jitsuyo Shinan Toroku Koho | 1996-2017 |
|---|---|---|---|
| Kokai Jitsuyo Shinan Koho | 1971-2017 | Toroku Jitsuyo Shinan Koho | 1994-2017 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 3014916 B2 (Kawasumi Laboratories, Inc.),<br>28 February 2000 (28.02.2000),<br>paragraphs [0019] to [0021]; fig. 5 to 6<br>(Family: none) | 1-2,4-7<br>3,8-15 |
| Y | JP 6-7432 A (Terumo Corp.),<br>18 January 1994 (18.01.1994),<br>paragraphs [0015] to [0038]; fig. 4<br>(Family: none) | 3,12-15 |
| Y | WO 2012/090834 A1 (Asahi Kasei Medical Co.,<br>Ltd.),<br>05 July 2012 (05.07.2012),<br>paragraphs [0048] to [0058]; fig. 5<br>& US 2012/0160763 A1<br>paragraphs [0054] to [0064]; fig. 5<br>& EP 2659917 A1 & CN 103313738 A | 8-11 |

☐ Further documents are listed in the continuation of Box C.    ☐ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 30 August 2017 (30.08.17) | 12 September 2017 (12.09.17) |

| Name and mailing address of the ISA/<br>Japan Patent Office<br>3-4-3,Kasumigaseki,Chiyoda-ku,<br>Tokyo 100-8915,Japan | Authorized officer<br><br><br>Telephone No. |
|---|---|

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3014916 B
- JP 3049182 B
- JP 3758853 B
- JP 4038547 B
- JP 3710384 B